# EUROPEAN PATENT SPECIFICATION

(11) **EP 2 761 299 B1**
(45) Date of publication and mention of the grant of the patent: **26.10.2016**
(21) Application number: 12775575.9
(22) Date of filing: 29.09.2012
(51) Int. Cl.: G01N 33/574, C12Q 1/68

(54) **MUTATIONS IN SF3B1 AND CHRONIC LYMPHOCYTIC LEUKEMIA**
SF3B1-MUTATIONEN UND CHRONISCHE LYMPHATISCHE LEUKÄMIE
MUTATIONS DANS SF3B1 ET LEUCÉMIE LYMPHOÏDE CHRONIQUE

(30) Priority: 29.09.2011 US 201161540618 P
(43) Date of publication of application: 06.08.2014
(73) Proprietor: Trovagene, Inc., San Diego, California 92121 (US)
(72) Inventor: ROSSI, Davide, San Diego California 92121 (US); GAIDANO, Gianluca, San Diego California 92121 (US); FOA, Robert, San Diego California 92121 (US)
(74) Representative: Lewis, Graham Matthew
(86) International application number: PCT/US2012/058164
(87) International publication number: WO 2013/049750

(56) References cited:
- WO-A2-2011/143656
- D. ROSSI ET AL: "Mutations of the SF3B1 splicing factor in chronic lymphocytic leukemia: association with progression and fludarabine-refractoriness", BLOOD, vol. 118, no. 26, 28 October 2011 (2011-10-28), pages 6904-6908, XP055052912, ISSN: 0006-4971, DOI: 10.1182/blood-2011-08-373159
- E PAPAEMMANUIL ET AL: "Somatic SF3B1 Mutation in Myelodysplasia with Ring Sideroblasts", THE NEW ENGLAND JOURNAL OF MEDICINE, vol. 365, 26 September 2011 (2011-09-26), pages 1384-1395, XP055053063, DOI: 10.1056/NEJMoa1103283)
- G. FABBRI ET AL: "Analysis of the chronic lymphocytic leukemia coding genome: role of NOTCH1 mutational activation", JOURNAL OF EXPERIMENTAL MEDICINE, vol. 208, no. 7, 13 June 2011 (2011-06-13) , pages 1389-1401, XP055053050, ISSN: 0022-1007, DOI: 10.1084/jem.20110921
- V VISCONTE ET AL: "SF3B1, a splicing factor is frequently mutated in refractory anemia with ring sideroblasts", LEUKEMIA, vol. 26, no. 3, 2 September 2011 (2011-09-02), pages 542-545, XP055053058, ISSN: 0887-6924, DOI: 10.1038/leu.2011.232

## Description

### FIELD OF THE DISCLOSURE

The present disclosure relates generally to the fields of molecular biology, genetics, and cancer. Specifically, mutations in the SF3B1 gene are used in *in vitro* methods to diagnose, prognose, and determine optimal treatment regimens for subjects with chronic lymphocytic leukemia.

### BACKGROUND OF THE DISCLOSURE

Chronic lymphocytic leukemia (CLL) is cancer of white blood cells called lymphocytes. As CLL progresses, the number of B lymphocytes or B cells present in the bone marrow increases. These cancerous B cells migrate or spread from the bone marrow into the blood. Via the blood these cancerous cells have access to all organs in the body. Most commonly, cancerous B cells affect lymph nodes, liver, and spleen. Ultimately the bone marrow fails to function properly, leading to death.

The clinical course of chronic lymphocytic leukemia (CLL) ranges from a very indolent disorder with a normal lifespan, to a rapidly progressive disease that ultimately becomes chemorefractory and leads to death. Occasionally, CLL undergoes histological transformation to Richter syndrome (RS).

The variable clinical course of CLL is driven, at least in part, by the molecular heterogeneity of the disease. Despite recent advances, the genetic lesions identified to date do not fully recapitulate CLL molecular pathogenesis and do not entirely explain the development of severe complications, such as chemorefractoriness and RS transformation, which still represent an unmet clinical need.

Identification of genetic lesions associated with chemorefractoriness represents a critical step for the early identification of high risk CLL patients and for the development molecularly tailored drugs.

Papaemmanuil et al (NEJM. 2011; 365(15); 1384-95) discloses a method for prognosing a subject with CLL by detecting mutations in the SF3B1 gene, which are indicative for a longer event-free survival.

### SUMMARY OF THE DISCLOSURE

The methods of the disclosure provide a solution to the long-felt and unsolved need for a biological indicator of disease progression and responsiveness to treatment. The disclosure provides missense and deletion mutations within the SF3B1 (splicing factor 3b, subunit 1, 155kDa) gene that change the amino acid sequence of the encoded protein. These changes in the protein have functional consequences. SF3B1 encodes subunit 1 of the splicing factor 3b protein complex. Under normal or wild-type conditions, splicing factor 3b, together with splicing factor 3a and a 12S RNA unit, forms the U2 small nuclear ribonucleoproteins complex (U2 snRNP). The splicing factor 3b/3a complex binds pre-mRNA. Splicing factor 3b is also a component of the minor U12-type spliceosome. Thus, subunit 1 of the splicing factor 3b protein complex plays a number of critical roles in the splicing mechanism of the cell. Mutations in SF3B1 affect the ability of a cell to convert pre- mRNA, which contains intronic sequence, into mature mRNA. In the context of CLL, these mutations are predictive of decreased survival in patients and increased resistance to treatment with fludarabine

The disclosure provides an *in vitro* method of prognosing a subject with chronic lymphocytic leukemia (CLL), comprising: (a) determining the sequence of a portion of the *SF3B1* gene in a biological sample obtained from the subject, wherein the portion of the *SF3B1* gene comprises a sequence that encodes for a HEAT3, HEAT4 or HEAT5 domain; and (b) analyzing the sequence of the *SF3B1* gene for a mutation, wherein the presence of the mutation in the sequence of the *SF3B1* gene predicts a decreased survival of the subject, thereby prognosing the subject with chronic lymphocytic leukemia (CLL). In many embodiments, the mutation is present within the HEAT3, HEAT4 or HEAT5 domain. In further embodiments, the method comprises preparation of a nucleic acid molecule from a subject followed by analysis as disclosed herein to detect nucleic acid alterations that predict or forecast the probable course and/or outcome of CLL.

Alternatively, or in addition, the disclosure provides an *in vitro* method of prognosing a subject with chronic lymphocytic leukemia (CLL), comprising: (a) determining the sequence of a portion of the SF3B1 polypeptide in a biological sample obtained from the subject, wherein the portion of the SF3B1 polypeptide comprises the sequence of a HEAT3, HEAT4 or HEAT5 domain; and (b) analyzing the sequence of the SF3B1 polypeptide for a mutation, wherein the presence of the mutation in the sequence of the SF3B1 polypeptide predicts a decreased survival of the subject, thereby prognosing the subject with chronic
lymphocytic leukemia (CLL). In many embodiments, the mutation is present within the HEAT3, HEAT4 or HEAT5 domain. In further embodiments, the method comprises preparation of a polypeptide molecule from a subject followed by analysis as disclosed herein to detect amino acid alterations that predict or forecast the probable course and/or outcome of CLL.

With respect to methods of prognosing a subject, the term decreased survival includes treatment-free survival or overall survival. Embodiments of the invention include predicting or forecasting the probable course and/or outcome of CLL in a subject with the nucleic acid, or amino acid, alteration in the absence of treatment for CLL. In other embodiments, the probable course and/or outcome is for a subject with the nucleic acid, or amino acid, alteration if treated with a disclosed treatment for CLL.

The disclosure also provides an *in vitro* method of determining the response of a subject with chronic lymphocytic leukemia (CLL) to treatment with fludarabine, comprising: (a) determining the sequence of a portion of the *SF3B1* gene in a biological sample obtained from the subject, wherein the portion of the *SF3B1* gene comprises a sequence that encodes for a HEAT3, HEAT4 or HEAT5 domain; and (b) analyzing the sequence of the *SF3B1* gene for a mutation, wherein the presence of the mutation in the sequence of the SF3B1 gene indicates that the subject is resistant or refractory to fludarabine, thereby determining the response of the subject with chronic lymphocytic leukemia (CLL) to treatment with fludarabine. In many embodiments, the mutation is present within the HEAT3, HEAT4 or HEAT5 domain. In further embodiments, the method comprises preparation of a nucleic acid molecule from a subject followed by analysis as disclosed herein to detect nucleic acid alterations that predict or forecast the probable non-responsiveness of CLL in the subject to treatment with fludarabine.

Alternatively, or in addition, the disclosure provides an *in vitro* method of determining the response of a subject with chronic lymphocytic leukemia (CLL) to treatment with fludarabine, comprising: (a) determining the sequence of a portion of the SF3B1 polypeptide in a biological sample obtained from the subject, wherein the portion of the SF3B1 polypeptide comprises a sequence of a HEAT3, HEAT4 or HEAT5 domain; and (b) analyzing the sequence of the SF3B1 polypeptide for a mutation, wherein the presence of the mutation in the sequence of the SF3B1 polypeptide indicates that the subject is resistant or refractory to fludarabine, thereby determining the response of the subject with chronic lymphocytic leukemia (CLL) to treatment with fludarabine. In many embodiments, the
mutation is present within the HEAT3, HEAT4 or HEAT5 domain. In further embodiments, the method comprises preparation of a polypeptide molecule from a subject followed by analysis as disclosed herein to detect amino acid alterations that predict or forecast the probable non-responsiveness of CLL in the subject to treatment with fludarabine.

In certain aspects of this method, the portion of the SF3B 1 polypeptide comprising the sequence of a HEAT3, HEAT4 or HEAT5 domain is selected from the group consisting of SEQ ID NO: 1, 2, 3, 4, 5, 6, 7, 8, 9, 10, 11, 12, 13, 14, 15, 16, and 19. In some embodiments, the portion of the SF3B1 polypeptide comprising the sequence of a HEAT3, HEAT4 or HEAT5 domain is SEQ ID NO:1 or 19.

With respect to methods of determining the response of a subject with chronic lymphocytic leukemia (CLL) to treatment with fludarabine, in subjects who carry one or more mutations in either the SF2B1 gene or polypeptide, the treatment with fludarabine is discontinued or replaced by treatment with chlorambucil, cyclophosphamide, rituximab, alemtuzumab, bendamustine, or a combination thereof. In many embodiments, the treatment with fludarabine is replaced by treatment with alemtuzumab. In other embodiments, treatment with fludarabine is not initiated, and instead, the subject is treated with chlorambucil, cyclophosphamide, rituximab, alemtuzumab, bendamustine, or a combination thereof. Therefore, the disclosure further includes a method of treating a subject with CLL comprising determination of responsiveness to fludarabine treatment as disclosed herein and discontinuing or altering the treatment as described above. In additional embodiments, the disclosure includes a method of treating a subject with CLL comprising determination of responsiveness to fludarabine treatment as disclosed herein and initiating treatment for the CLL with a therapy other than fludarabine as described above.

With respect to any method of the disclosure, the analyzing step may include polymerase chain reaction (PCR), Sanger sequencing, next generation sequencing, or a combination thereof as known to the skilled person. In some embodiments, the analysis may use prepared or isolated DNA molecules that are used as templates or for hybridization. In some cases, a DNA molecule as template is amplified, such as by PCR or quantitative PCR, with optional detectable labeling of the amplified molecules to aid in their detection. In other cases, the amplified molecules may be detected based upon hybridization to a polynucleotide probe. In alternative cases, a prepared or isolated DNA molecule is not copied or amplified, but directly sequenced instead, with optional direct or indirect immobilization on a solid support or solid phase medium, prior to sequencing. Non-limiting examples of a solid support or solid phase medium include a bead, a microbead, or other insoluble material. In further cases, a DNA molecule may be prepared or isolated by incorporation into, or as part of, an emulsion or a compartment, such as a droplet or microdroplet, or other suspension in solution.

In the practice of the disclosure, the subject may be diagnosed with chronic lymphocytic leukemia (CLL) or may not be diagnosed with chronic lymphocytic leukemia (CLL). These methods can be applied at any point in the diagnosis or treatment of a subject. Subjects with CLL may present one or more of the following non-limiting list of symptoms: enlarged lymph nodes, liver, or spleen; excessive sweating or night sweats; fatigue; fever; recurring infections; and unintentional weight loss. Subjects with CLL may present a higher-than-normal white blood cell count, anemia and/or thrombocytopenia. Subject may be treated with one or more of the following non-limiting exemplary treatments: fludarabine (Fludara), chlorambucil, cyclophosphamide (Cytoxan), rituximab (Rituxan), alemtuzumab (Campath), bendamustine, or a combination thereof. Subjects who are resistant or refractory to treatment with fludarabine because they carry one or more of the mutations described herein may be treated with Alemtuzumab (Campath). Subjects who carry one or more of the mutations described herein may be also treated with Bendamustine, particularly when the CLL returns after an initial treatment or when the subject or patient suffers a relapse.

In the practice of the disclosure, the biological sample includes an isolated and purified genomic DNA, cDNA, or RNA molecule. Alternatively, the biological sample includes an isolated and purified polypeptide molecule. The biological sample can be obtained from one or more tissues or bodily fluids. For mutation detection, exemplary tissues include, but are not limited to, bone marrow, blood cells, peripheral blood cells, lymph nodes, spleen, muscle tissue (including, smooth, visceral, striated, skeletal, or cardiac muscles composed of muscle cells or fibers), nervous system tissue (including, but not limited to, the neurons and glia or the central and peripheral nervous system), or epithelial tissues (including, but not limited to, epithelial cells that comprise the skin, respiratory tract, reproductive tract, and digestive tract). To confirm the somatic origin of the mutations, exemplary specimens include saliva, epidermal cells obtained by a non-invasive scraping of the skin or a swab of the inner cheek. In some embodiments, the tissue sample comprises red or white blood cells isolated from whole blood. Exemplary bodily fluids include, but are not limited to, aqueous humour, vitreous humour, bile, whole blood, blood serum, breast milk, cerebrospinal fluid (CSF), endolymph, perilymph, gastric juice, mucus (including nasal drainage and phlegm), peritoneal fluid, pleural fluid, saliva, sebum (skin oil), sweat, tears, and urine. In some embodiments, the bodily fluid is whole blood, blood serum, endolymph, perilymph, saliva, or urine.

In the practice of the disclosure, the SF3B1 mutation may be a missense mutation or an in-frame deletion in the polynucleotide or polypeptide sequence in a *SF3B1* gene or the resultant polypeptide encoded by a *SF3B1* gene, respectively. In many embodiments, a missense mutation or an in-frame deletion is in a HEAT3, HEAT4, or HEAT5 domain.

In some disclosed methods of prognosing a subject, the mutation may be a substitution of a Guanine (G) for an Adenine (A) at nucleotide base position 2044 or 2146; a substitution of a Thymine (T) for a Guanine (G) at nucleotide base position 2046; or a substitution of an Alanine (A) for a Guanine (G) at nucleotide base position 2267, of SEQ ID NO: 17 (Genbank Accession No. NM_012433.2).

In other disclosed methods of prognosing a subject, the mutation may be a substitution of a Glutamic Acid (Glu or E) residue for a Lysine (Lys or K) residue at codon 666 or 700; results in a substitution of an Asparagine (Asn or N) residue for a Lysine (Lys or K) residue at codon 666; or a substitution of a Glutamic Acid (Glu or E) residue for a Glycine (Gly or G) residue at codon 740, of SEQ ID NO: 19 (Genbank Accession No. NP_036565.2).

In some disclosed methods of determining the response of a subject to treatment with fludarabine, the mutation may be a substitution of a Thymine (T) for an Adenine (A) at nucleotide base position 1938; a substitution of an Adenine (A) for a Cytosine (C) at nucleotide base position 2034; a substitution of a Guanine (G) for a Cytosine (C) at nucleotide base position 2032; a substitution of a Guanine (G) for an Adenine (A) at nucleotide base position 2044; a substitution of a Guanine (G) for an Adenine (A) at nucleotide base position 2146; a deletion of the nucleotide sequence CAGAAA corresponding to nucleotide base positions 2143 to 2148; or a substitution of a Guanine (G) for a Cytosine (C) at nucleotide base position 2056, of SEQ ID NO: 17.

In some disclosed methods of determining the response of a subject to treatment with fludarabine, the mutation may be a substitution of a Serine (Ser or S) residue for an Arginine (Arg or R) residue at codon 630; a substitution of a Glutamine (Gln or Q) residue for a Histidine (His or H) residue at codon 662; a substitution of an Aspartic Acid (Asp or D) residue for a Histidine (His or H) residue at codon 662; a substitution of a Glutamic Acid (Glu or E) residue for a Lysine (Lys or K) residue at codon 666 or 700; a deletion of a Glutamine (Gln or Q) residue at codon 699 and a Lysine (Lys or K) residue at codon 700; a substitution of a Glutamic Acid (Glu or E) residue for a Glutamine (Gln or Q) residue at codon 670, of SEQ ID NO: 19.

In many disclosed methods of prognosing a subject, the mutation results in a substitution of a Glutamic Acid (Glu or E) residue for a Lysine (Lys or K) residue at codon 666 or 700. When either the K666E or K700E substitution is present, the mutation is a substitution of a Guanine (G) for an Adenine (A) at nucleotide base position 2044 or 2146, respectively. With respect to some disclosed methods of prognosing a subject, the mutation results in a substitution of an Asparagine (Asn or N) residue for a Lysine (Lys or K) residue at codon 666. When the K666N substitution is present, the mutation is a substitution of a Thymine (T) for a Guanine (G) at nucleotide base position 2046. With respect to other disclosed methods of prognosing a subject, the mutation results in a substitution of a Glutamic Acid (Glu or E) residue for a Glycine (Gly or G) residue at codon 740. When the G740E substitution is present, the mutation is a substitution of an Adenine (A) for a Guanine (G) at nucleotide base position 2267.

In other disclosed methods of determining the response of a subject to treatment with fludarabine, the mutation results in a substitution of a Serine (Ser or S) residue for an Arginine (Arg or R) residue at codon 630. When the R630S substitution is present, the mutation is a substitution of a Thymine (T) for an Adenine (A) at nucleotide base position 1938. In some disclosed methods of determining the response of a subject to treatment with fludarabine, the mutation results in a substitution of a Glutamine (Gln or Q) residue for a Histidine (His or H) residue at codon 662. When the H662Q substitution is present, the mutation is a substitution of an Adenine (A) for a Cytosine (C) at nucleotide base position 2034. In further disclosed methods of determining the response of a subject to treatment with fludarabine, the mutation results in a substitution of an Aspartic Acid (Asp or D) residue for a Histidine (His or H) residue at codon 662. When the H662D substitution is present, the mutation is a substitution of a Guanine (G) for a Cytosine (C) at nucleotide base position 2032. In additional disclosed methods of determining the response of a subject to treatment with fludarabine, the mutation results in a substitution of a Glutamic Acid (Glu or E) residue for a Lysine (Lys or K) residue at codon 666 or 700. When the K666E substitution is present, the mutation is a substitution of a Guanine (G) for an Adenine (A) at nucleotide base position 2044. When the K700E substitution is present, the mutation is a substitution of a Guanine (G) for an Adenine (A) at nucleotide base position 2146. In cases of disclosed methods of determining the response of a subject to treatment with fludarabine, the mutation results in a deletion of a Glutamine (Gln or Q) residue at codon 699 and a Lysine (Lys or K) residue at codon 700. When the Q699-K700 deletion is present, the mutation is a deletion of the nucleotide sequence CAGAAA corresponding to nucleotide base positions 2143 to 2148. In further cases of disclosed methods of determine the response of a subject to treatment with fludarabine, the mutation results in a substitution of a Glutamic Acid (Glu or E) residue for a Glutamine (Gln or Q) residue at codon 670. When the Q670E substitution is present, the mutation is a substitution of a Guanine (G) for a Cytosine (C) at nucleotide base position 2056.

Homo sapiens splicing factor 3b, subunit 1, 155kDa, *SF3B1,* transcript variant 1, is encoded by the following mRNA sequence (NM_012433.2, SEQ ID NO: 17) (portion encoding HEAT3, HEAT4, and HEAT5 domain is underlined):

1 ggaagttctt gggagcgcca gttccgtctg tgtgttcgag tggacaaaat ggcgaagatc
61 gccaagactc acgaagatat tgaagcacag attcgagaaa ttcaaggcaa gaaggcagct
121 cttgatgaag ctcaaggagt gggcctcgat tctacaggtt attatgacca ggaaatttat
181 ggtggaagtg acagcagatt tgctggatac gtgacatcaa ttgctgcaac tgaacttgaa
241 gatgatgacg atgactattc atcatctacg agtttgcttg gtcagaagaa gccaggatat
301 catgcccctg tggcattgct taatgatata ccacagtcaa cagaacagta tgatccattt
361 gctgagcaca gacctccaaa gattgcagac cgggaagatg aatacaaaaa gcataggcgg
421 accatgataa tttccccaga gcgtcttgat ccttttgcag atggagggaa aacccctgat
481 cctaaaatga atgctaggac ttacatggat gtaatgcgag aacaacactt gactaaagaa
541 gaacgagaaa ttaggcaaca gctagcagaa aaagctaaag ctggagaact aaaagtcgtc
601 aatggagcag cagcgtccca gcctccatca aaacgaaaac ggcgttggga tcaaacagct
661 gatcagactc ctggtgccac tcccaaaaaa ctatcaagtt gggatcaggc agagacccct
721 gggcatactc cttccttaag atgggatgag acaccaggtc gtgcaaaggg aagcgagact
781 cctggagcaa ccccaggctc aaaaatatgg gatcctacac ctagccacac accagcggga
841 gctgctactc ctggacgagg tgatacacca ggccatgcga caccaggcca tggaggcgca
901 acttccagtg ctcgtaaaaa cagatgggat gaaaccccca aaacagagag agatactcct
961 gggcatggaa gtggatgggc tgagactcct cgaacagatc gaggtggaga ttctattggt
1021 gaaacaccga ctcctggagc cagtaaaaga aaatcacggt gggatgaaac accagctagt
1081 cagatgggtg gaagcactcc agttctgacc cctggaaaga caccaattgg cacaccagcc
1141 atgaacatgg ctacccctac tccaggtcac ataatgagta tgactcctga acagcttcag
1201 gcttggcggt gggaaagaga aattgatgag agaaatcgcc cactttctga tgaggaatta
1261 gatgctatgt tcccagaagg atataaggta cttcctcctc cagctggtta tgttcctatt
1321 cgaactccag ctcgaaagct gacagctact ccaacacctt tgggtggtat gactggtttc
1381 cacatgcaaa ctgaagatcg aactatgaaa agtgttaatg accagccatc tggaaatctt
1441 ccatttttaa aacctgatga tattcaatac tttgataaac tattggttga tgttgatgaa
1501 tcaacactta gtccagaaga gcaaaaagag agaaaaataa tgaagttgct tttaaaaatt
1561 aagaatggaa caccaccaat gagaaaggct gcattgcgtc agattactga taaagctcgt
1621 gaatttggag ctggtccttt gtttaatcag attcttcctc tgctgatgtc tcctacactt
1681 gaggatcaag agcgtcattt acttgtgaaa gttattgata ggatactgta caaacttgat
1741 gacttagttc gtccatatgt gcataagatc ctcgtggtca ttgaaccgct attgattgat
1801 gaagattact atgctagagt ggaaggccga gagatcattt ctaatttggc aaaggctgct
1861 ggtctggcta ctatgatctc taccatgaga cctgatatag ataacatgga tgagtatgtc
1921 cgtaacacaa cagctagagc ttttgctgtt gtagcctctg ccctgggcat tccttcttta
1981 ttgcccttct taaaagctgt gtgcaaaagc aagaagtcct ggcaagcgag acacactggt
2041 attaagattg tacaacagat agctattctt atgggctgtg ccatcttgcc acatcttaga
2101 agtttagttg aaatcattga acatggtctt gtggatgagc agcagaaagt tcggaccatc
2161 agtgctttgg ccattgctgc cttggctgaa gcagcaactc cttatggtat cgaatctttt
2221 gattctgtgt taaagccttt atggaagggt atccgccaac acagaggaaa gggtttggct
2281 gctttcttga aggctattgg gtatcttatt cctcttatgg atgcagaata tgccaactac
2341 tatactagag aagtgatgtt aatccttatt cgagaattcc agtctcctga tgaggaaatg

2401 aaaaaaattg tgctgaaggt ggtaaaacag tgttgtggga cagatggtgt agaagcaaac
2461 tacattaaaa cagagattct tcctcccttt tttaaacact tctggcagca caggatggct
2521 ttggatagaa gaaattaccg acagttagtt gatactactg tggagttggc aaacaaagta
2581 ggtgcagcag aaattatatc caggattgtg gatgatctga aagatgaagc cgaacagtac
2641 agaaaaatgg tgatggagac aattgagaaa attatgggta atttgggagc agcagatatt
2701 gatcataaac ttgaagaaca actgattgat ggtattcttt atgctttcca agaacagact
2761 acagaggact cagtaatgtt gaacggcttt ggcacagtgg ttaatgctct tggcaaacga
2821 gtcaaaccat acttgcctca gatctgtggt acagttttgt ggcgtttaaa taacaaatct
2881 gctaaagtta ggcaacaggc agctgacttg atttctcgaa ctgctgttgt catgaagact
2941 tgtcaagagg aaaaattgat gggacacttg ggtgttgtat tgtatgagta tttgggtgaa
3001 gagtaccctg aagtattggg cagcattctt ggagcactga aggccattgt aaatgtcata
3061 ggtatgcata agatgactcc accaattaaa gatctgctgc ctagactcac ccccatctta
3121 aagaacagac atgaaaaagt acaagagaat tgtattgatc ttgttggtcg tattgctgac
3181 aggggagctg aatatgtatc tgcaagagag tggatgagga tttgctttga gcttttagag
3241 ctcttaaaag cccacaaaaa ggctattcgt agagccacag tcaacacatt tggttatatt
3301 gcaaaggcca ttggccctca tgatgtattg gctacacttc tgaacaacct caaagttcaa
3361 gaaaggcaga acagagtttg taccactgta gcaatagcta ttgttgcaga aacatgttca
3421 ccctttacag tactccctgc cttaatgaat gaatacagag ttcctgaact gaatgttcaa
3481 aatggagtgt taaaatcgct ttccttcttg tttgaatata ttggtgaaat gggaaaagac
3541 tacatttatg ccgtaacacc gttacttgaa gatgctttaa tggatagaga ccttgtacac
3601 agacagacgg ctagtgcagt ggtacagcac atgtcacttg gggtttatgg atttggttgt
3661 gaagattcgc tgaatcactt gttgaactat gtatggccca atgtatttga gacatctcct
3721 catgtaattc aggcagttat gggagcccta gagggcctga gagttgctat tggaccatgt
3781 agaatgttgc aatattgttt acagggtctg tttcacccag cccggaaagt cagagatgta
3841 tattggaaaa tttacaactc catctacatt ggttcccagg acgctctcat agcacattac
3901 ccaagaatct acaacgatga taagaacacc tatattcgtt atgaacttga ctatatctta
3961 taattttatt gtttattttg tgtttaatgc acagctactt cacaccttaa acttgctttg
4021 atttggtgat gtaaactttt aaacattgca gatcagtgta gaactggtca tagaggaaga
4081 gctagaaatc cagtagcatg atttttaaat aacctgtctt tgtttttgat gttaaacagt
4141 aaatgccagt agtgaccaag aacacagtga ttatatacac tatactggag ggatttcatt
4201 tttaattcat ctttatgaag atttagaact cattccttgt gtttaaaggg aatgtttaat
4261 tgagaaataa acatttgtgt acaaaatgct aaaaaaaaaa aaaaaaaaaa aaaa

Homo sapiens splicing factor 3b, subunit 1, 155kDa, *SF3B1,* transcript variant 2, is encoded by the following mRNA sequence (NM_001005 526, SEQ ID NO: 18):

1 ggaagttctt gggagcgcca gttccgtctg tgtgttcgag tggacaaaat ggcgaagatc
61 gccaagactc acgaagatat tgaagcacag attcgagaaa ttcaaggcaa gaaggcagct
121 cttgatgaag ctcaaggagt gggcctcgat tctacaggtt attatgacca ggaaatttat
181 ggtggaagtg acagcagatt tgctggatac gtgacatcaa ttgctgcaac tgaacttgaa
241 gatgatgacg atgactattc atcatctacg agtttgcttg gtcagaagaa gccaggatat
301 catgcccctg tggcattgct taatgatata ccacagtcaa cagaacagta tgatccattt
361 gctgagcaca gacctccaaa gattgcagac cgggaagatg aatacaaaaa gcataggcgg
421 accatgataa tttccccaga gcgtcttgat ccttttgcag atggcttcta ttctgctgct
481 tgaagtcaga actgctgatg gagacaaagg cacgaaagtg tacgtattcc ggattagcaa
541 cccaggaacc catcacttct gaagactcta aactgtgctg tcattttgtt tttatatgca
601 ttaaaatatt tgttttaaaa aaaaaaaaaa aaaaaaaaaa aaaaaaa

Homo sapiens splicing factor 3b, subunit 1, 155kDa, *SF3B1,* transcript variant 1, is encoded by the following amino acid sequence (NP_036565.2, SEQ ID NO: 19) (portion containing HEAT3, HEAT4, and HEAT5 domain is underlined):

  1 makiakthed ieaqireiqg kkaaldeaqg vgldstgyyd qeiyggsdsr fagyvtsiaa
 61 teledddddy ssstsllgqk kpgyhapval lndipqsteq ydpfaehrpp kiadredeyk
121 khrrtmiisp erldpfadgg ktpdpkmnar tymdvmreqh ltkeereirq qlaekakage
181 lkvvngaaas qppskrkrrw dqtadqtpga tpkklsswdq aetpghtpsl rwdetpgrak
241 gsetpgatpg skiwdptpsh tpagaatpgr gdtpghatpg hggatssark nrwdetpkte
301 rdtpghgsgw aetprtdrgg dsigetptpg askrksrwde tpasqmggst pvltpgktpi

361 gtpamnmatp tpghimsmtp eqlqawrwer eidernrpls deeldamfpe gykvlpppag
421 yvpirtpark ltatptplgg mtgfhmqted rtmksvndqp sgnlpflkpd diqyfdkllv
481 dvdestlspe eqkerkimkl llkikngtpp mrkaalrqit dkarefgagp lfnqilpllm
541 sptledqerh llvkvidril ykldd lvrpy vhkilvviep llidedyyar vegreiisnl
601 akaaglatmi stmrpdidnm deyvrnttar afavvasalg ipsllpflka vckskkswqa
661 rhtgikivqq iailmgcail phlrslveii ehglvdeqqk vrtisalaia alaeaatpyg
721 iesfdsvlkp lwkgirqhrg kglaaflkai gyliplmdae yanyytrevm lilirefqsp
781 deemkkivlk vvkqccgtdg veanyiktei lppffkhfwq hrmaldrrny rqlvdttvel
841 ankvgaaeii srivddlkde aeqyrkmvme tiekimgnlg aadidhklee qlidgilyaf
901 qeqttedsvm lngfgtvvna lgkrvkpylp qicgtvlwrl nnksakvrqq aadlisrtav
961 vmktcqeekl mghlgvvlye ylgeeypevl gsilgalkai vnvigmhkmt ppikdllprl
1021 tpilknrhek vqencidlvg riadrgaeyv sarewmricf ellellkahk kairratvnt
1081 fgyiakaigp hdvlatllnn lkvqerqnrv cttvaiaiva etcspftvlp almneyrvpe
1141 lnvqngvlks lsflfeyige mgkdyiyavt plledalmdr dlvhrqtasa vvqhmslgvy
1201 gfgcedslnh llnyvwpnvf etsphviqav mgaleglrva igpcrmlqyc lqglfhpark
1261 vrdvywkiyn siyigsqdal iahypriynd dkntyiryel dyil

Homo sapiens splicing factor 3b, subunit 1, 155kDa, *SF3B1,* transcript variant 2, is encoded by the following amino acid sequence (NP_001005526, SEQ ID NO: 20):

  1 makiakthed ieaqireiqg kkaaldeaqg vgldstgyyd qeiyggsdsr fagyvtsiaa
 61 teledddddy ssstsllgqk kpgyhapval lndipqsteq ydpfaehrpp kiadredeyk
121 khrrtmiisp erldpfadgf ysaa

In some non-limiting embodiments of the disclosure, a disclosed method may be used *in vitro* to analyze SF3B1 sequences and sequence alterations as disclosed herein without including a act of diagnosis or medical treatment.

Other features and advantages of the disclosure will be apparent from and are encompassed by the following detailed description and claims.

### BRIEF DESCRIPTION OF THE DRAWINGS

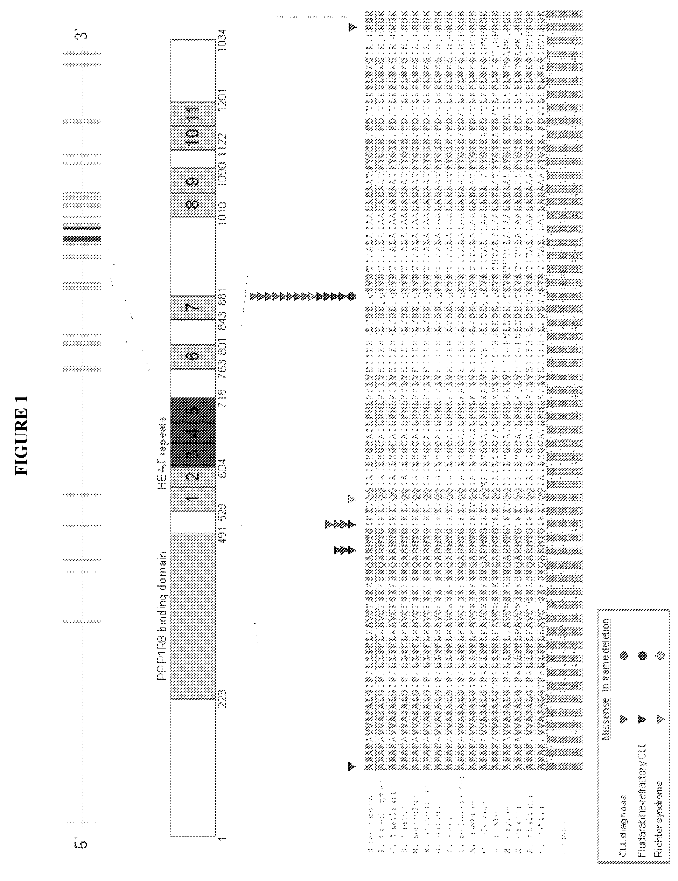

Figure 1 is a schematic diagram of the human *SF3B1* gene (top) and protein (bottom) with its functional domains (PPP1R8 binding domain and HEAT repeats). A corresponding multiple alignment of the HEAT3, HEAT4 and HEAT5 amino acid sequences of the human SF3B1 protein with orthologous SF3B1 proteins (n=15) is provided. Amino acids conserved among species are highlighted (grey). Color-coded shapes indicate the position of the mutations found in CLL at diagnosis (green, missense mutations corresponding to K666E, K700E, and G740E; also see Table 4), in fludarabine-refractory CLL (red, missense mutations corresponding to R630S, H662Q, H662D, K666E, and K700E; in-frame deletions corresponding to de1Q699_K700; also see Table 4), and in Richter syndrome (RS) (orange, missense mutations corresponding to Q670E and K700E within; also see Table 4). The following sequences are provided: 
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK
 (*Homo Sapiens*, SEQ ID NO: 1), 
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (P.
troglodytes, SEQ ID NO: 2),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (C.
familiaris, SEQ ID NO: 3),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (B.
tauris, SEQ ID NO: 4),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (M.
musculus, SEQ ID NO: 5),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (R.
norvegicus, SEQ ID NO: 6),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (G.
gallus, SEQ ID NO: 7),
ARAFAVVASALGIPSLLPFLKAVCKSKKSWQARHTGIKIVQQIAILMGCAILPHLRSL
VEIIEHGLVDEQQKVRTISALAIAALAEAATPYGIESFDSVLKPLWKGIRQHRGK (D.
rerio, SEQ ID NO: 8),
ARAFAVVASALGIPSLLPFLKAVCKSKKSWQARHTGIKIVQQIAILMGCAILPHLKAL
VEIIEHGLVDEQQKVRTITALAIAALAEAATPYGIESFDSVLKPLWKGIRTHRGK (D.
melanogaster, SEQ ID NO: 9),
ARAFAVVASALGIPSLLPFLKAVCKSKKSWQARHTGIKIVQQIAILMGCAILPHLKSL
VEIIEHGLVDEQQKVRTITALALAALAEAATPYGIESFDSVLKPLWKGIRTHRGK (A.
gambiae, SEQ ID NO: 10),
ARAFAVVASALGIPALLPFLKAVCKSKKSWQARHTGIKIVQQMAILMGCAVLPHLK
ALVDIVESGLDDEQQKVRTITALCLAALAEASSPYGIEAFDSVLKPLWKGIRMHRGK
(C. elegans, SEQ ID NO: 11),
ARAFSVVASALGVPALLPFLKAVCRSKKSWQARHTGVRIIQQIALLLGCSILPHLKNL
VDCIGHGLEDEQQKVRIMTALSLSALAEAATPYGIEAFDSVLKPLWSGVQRHRGK (S.
 
Pombe, SEQ ID NO: 12),
ARAFAVVASALGIPALLPFLQAVCRSKKSWQARHTGVKIVQQIPILMGCAVLPHLKR
LVDCIGPNLNDEQTKVRTVTSLAIAALAEAANPYGIESFDDILNPLWTGARKQRGK
(M. oryzae, SEQ ID NO: 13),
ARAFAVVASALGIPALLPFLRAVCRSKKSWQARHTGVKIVQQIPILMGCAVLPHLKQ
LVDCIGPNLNDEQTKVRTVTSLAIAALAEASNPYGIESFDDILNPLWTGARKQRGK
(M. crassa, SEQ ID NO: 14),
ARAFSVVASALGIPALLPFLKAVCQSKRSWQARHTGIKIVQQIAILIGCAVLPHLRSLV
EIIEHGLSDENQKVRTITALSLAALAEAAAPYGIESFDSVLKPLWKGIRSHRGK (A.
thaliana, SEQ ID NO: 15), and
ARAFSVVASALGTPALLPFLKAVCQSKKSWQARHTGIKIVQQIAILMGCAVLPHLKS
LVEIIEHGLSDENQKVRTITALSLATLAEAAAPYGIESFDTVLKPLWKGIRSHRGK (O.
Sativa, SEQ ID NO: 16).

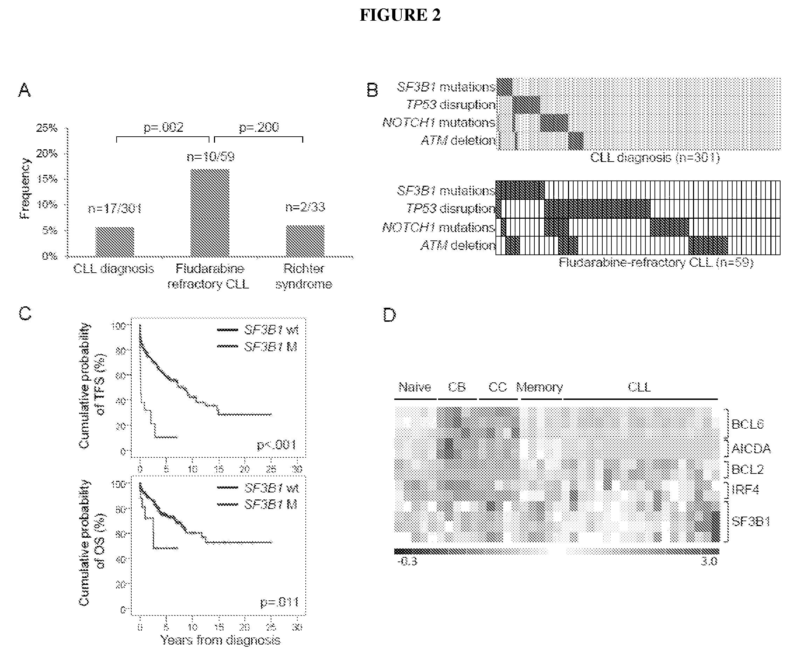

Figure 2A-D is a series of graphs depicting the prevalence, mutual relationship with other genetic lesions, and clinical impact of *SF3B1* mutations in CLL. Panel A: prevalence of *SF3B1* mutations in CLL at diagnosis, in fludarabine-refractory CLL, and in Richter syndrome; numbers on top indicate the actual number of mutated samples over the total number analyzed. Panel B: mutual relationship of *SF3B1* mutations with other genetic lesions in CLL at diagnosis and in fludarabine-refractory CLL. In the heat map, rows correspond to identical genes, and columns represent individual patients color-coded based on the gene status (white: wild type; red: mutations of *SF3B1 ,* mutations of *NOTCH1,* mutations and/or deletion of *TP53,* deletion of ATM). Panel C: Kaplan-Meier estimates of treatment-free survival (TFS) and overall survival (OS) from diagnosis in the consecutive series of newly diagnosed and previously untreated CLL (n=301). *SF3B1* wild type (*SF3B1* wt) is represented by the blue line. *SF3B1* mutated cases (SF3B1 M) are represented by the red line. Panel D: Gene expression levels of *BCL6, AICDA, BCL2, IRF4* and *SF3B1* in normal B-cell subpopulations (Naive; Centroblasts, CB; Centrocytes, CC; Memory) and CLL samples. Relative levels of gene expression are depicted with a color scale: red represents the highest level of expression and blue represents the lowest level.

### DETAILED DESCRIPTION OF MODES OF PRACTICING THE DISCLOSURE

The genetic lesions identified in chronic lymphocytic leukemia (CLL) do not entirely recapitulate the disease pathogenesis and the development of serious complications, such as chemorefractoriness. While investigating the coding genome of fludarabine-refractory CLL, it was discovered that mutations of SF3B1, encoding a splicing factor and representing a critical component of the cell spliceosome, were recurrent in 10/59 (17%) fludarabine-refractory cases, with a frequency significantly higher than that observed in a consecutive CLL cohort sampled at diagnosis (17/301, 5%; p=.002). Mutations were somatically acquired, were generally represented by missense nucleotide changes, clustered in selected HEAT repeats of the SF3B1 protein, recurrently targeted three hotspots (codons 662, 666 and 700), and predicted poor prognosis. In fludarabine-refractory CLL, SF3B 1 mutations and TP53 disruption distributed in a mutually-exclusive fashion (p=.046). Identification of SF3B1 mutations indicates that splicing regulation is a novel pathogenetic mechanism of clinical relevance in CLL.

The clinical course of chronic lymphocytic leukemia (CLL) ranges from a very indolent disorder with a normal lifespan, to a rapidly progressive disease leading to death. Occasionally, CLL undergoes histological transformation to Richter syndrome (RS) (Müller-Hermelink HK et al. In: Swerdlow SH et al. eds. World Health Organization Classification of Tumours, Pathology and Genetics of Tumours of Haematopoietic and Lymphoid Tissues. Lyon, France: IARC; 2008:180-182; HallekM, et al. Blood. 2008; 111(12):5446-5456; Rossi D, et al. Blood. 2011; 117(12):3391-3401). The variable clinical course of CLL is driven, at least, in part, by the immunogenetic and molecular heterogeneity of the disease (Chiorazzi N, et al. N Engl J Med. 2005; 352(8):804-815).

Despite recent advances, the genetic lesions identified to date do not fully recapitulate CLL molecular pathogenesis and do not entirely explain the development of severe complications, such as chemorefractoriness, which still represent an unmet clinical need (Kay NE, et al. Leukemia 2007; 21(9):1885-1891). Fludarabine-refractoriness is due to TP53 disruption in ~40% of refractory cases, but in a sizeable fraction of patients the molecular basis of this aggressive clinical phenotype remains unclear (Stilgenbauer S and Zenz T. Hematology Am Soc Hematol Educ Program. 2010; 2010:481-488).

Recently, two independent studies of the CLL coding genome investigated at disease presentation have revealed a restricted number of mutated genes, including *NOTCH1* (Fabbri G, et al. J Exp Med. 2011; 208(7):1389-1401; Puente XS, et al. Nature. 2011; 475(7354):101-105). These studies have provided a proof of concept that, similar to other malignancies, genome-wide mutational analysis might identify novel lesions of potential biological and clinical relevance in CLL. Following initial findings from whole exome sequencing of the coding genome of fludarabine-refractory CLL, the occurrence of recurrent mutations of *SF3B1 ,* a critical component of the cell spliceosome, is disclosed herein.

The terms "nucleic acid" and "polynucleotide" are used interchangeably herein to refer to single- or double-stranded RNA, DNA, or mixed polymers. Polynucleotides may include genomic sequences, extra-genomic and plasmid sequences, and smaller engineered gene segments that express, or may be adapted to express polypeptides.

An "isolated nucleic acid" is a nucleic acid that is substantially separated from other genome DNA sequences as well as proteins or complexes such as ribosomes and polymerases, which naturally accompany a native sequence. The term embraces a nucleic acid sequence that has been removed from its naturally occurring environment, and includes recombinant or cloned DNA isolates and chemically synthesized analogues or analogues biologically synthesized by heterologous systems. A substantially pure nucleic acid includes isolated forms of the nucleic acid. This refers to the nucleic acid as originally isolated and does not exclude genes or sequences later added to the isolated nucleic acid by the hand of man. In addition to preparation of nucleic acid molecules as described above, the disclosure includes preparation of nucleic acid molecules by direct or indirect immobilization on a solid support or solid phase medium. Direct immobilization may be mediated by hydrogen bonds, such as in the case of hybridization as a non-limiting example, or be mediated by one or more covalent bonds. Non-limiting examples include hybridization of nucleic acid molecules to a polynucleotide probe on a microarray or a bead or another solid support to detect a nucleic acid molecule of interest. Optionally, the hybridized nucleic acid molecules may be those amplified by PCR. Indirect immobilization of a nucleic acid molecule may be mediated by binding to an immobilized polymerase, such as an RNA polymerase or DNA polymerase. In additional embodiments, a nucleic acid molecule may be prepared for sequencing by ligation to a known nucleic acid sequence or binding to a primer polynucleotide by basepair complementarity. In some embodiments, an immobilized nucleic acid molecule may be sequenced without need for amplification or replication. In further embodiments, a prepared nucleic acid molecule may be an RNA molecule that has been detectably labeled to aid in its analysis or an RNA molecule that has been coverted into a cDNA molecule for use as described herein.

The term "polypeptide" is used in its conventional meaning, *i.e.,* as a sequence of amino acids. The polypeptides are not limited to a specific length of the product. Peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof.

An "isolated polypeptide" is one that has been identified and separated and/or recovered from a component of its natural environment. In some embodiments, the isolated polypeptide will be purified (1) to greater than 95% by weight of polypeptide as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated polypeptide includes the polypeptide *in situ* within recombinant cells since at least one component of the polypeptide's natural environment will not be present. Ordinarily, however, isolated polypeptide will be prepared by at least one purification step.

A "native sequence" polynucleotide is one that has the same nucleotide sequence as a polynucleotide derived from nature. A "native sequence" polypeptide is one that has the same amino acid sequence as a polypeptide (*e.g.,* protein subunit) derived from nature (*e.g.,* from any species). Such native sequence polynucleotides and polypeptides can be isolated from nature or can be produced by recombinant or synthetic means.

A "mutant or mutated" polynucleotide, as the term is used herein, is a polynucleotide that typically differs from a polynucleotide specifically disclosed herein in one or more substitutions, deletions, additions and/or insertions. Such variants may be naturally occurring or may be synthetically generated, for example, by modifying one or more of the polynucleotide sequences of the disclosure and evaluating one or more biological activities of the encoded polypeptide as described herein and/or using any of a number of techniques well known in the art. "Mutant" polynucleotides of the disclosure contain one or more substitutions, deletions, additions and/or insertions that alter the function of the resultant polypeptide encoded therein. Alternatively, or in addition, "modified" polynucleotides of the disclosure contain one or more substitutions, deletions, additions and/or insertions that do not alter the function of the resultant polypeptide encoded therein. In some embodiments, a "mutant or mutated" polynucleotide is defined by reference to a wildtype sequence as disclosed herein. Additionally, a "mutant or mutated" polynucleotide may be prepared, and optionally detected, in the same manner as other polynucleotides disclosed herein.

A "mutant or mutated" polypeptide, as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences of the disclosure and evaluating one or more biological activities of the polypeptide as described herein and/or using any of a number of techniques well known in the art. "Mutant" polypeptides of the disclosure contain one or more substitutions, deletions, additions and/or insertions that alter the function of the resultant polypeptide. Alternatively, or in addition, "modified" polypeptides of the disclosure contain one or more substitutions, deletions, additions and/or insertions that do not alter the function of the resultant polypeptide. In some embodiments, a "mutant or mutated" polypeptide is defined by reference to a wildtype sequence as disclosed herein. Additionally, a "mutant or mutated" polypeptide may be analyzed or detected by any method known to the skilled person. Non-limiting examples include peptide sequencing, analysis by mass spectroscopy, and binding by antibodies or receptors.

Modifications may be made in the structure of the wild type or mutant polynucleotides and polypeptides of the present disclosure and still obtain a functional molecule that encodes a variant or derivative polypeptide with desirable characteristics. When it is desired to alter the amino acid sequence of a polypeptide to create an equivalent, or even an improved, variant or portion of a polypeptide of the disclosure, one skilled in the art will typically change one or more of the codons of the encoding DNA sequence.

For example, certain amino acids may be substituted for other amino acids in a protein structure without appreciable loss of its ability to bind other polypeptides or cells. Because it is the binding capacity and nature of a protein that defines that the biological functional activity of a protein, certain amino acid sequence substitutions can be made in a protein sequence, and, of course, the underlying DNA coding sequence of the protein, and nevertheless obtain a protein with like properties. It is thus contemplated that various changes may be made in the peptide sequences of the disclosed compositions, or corresponding DNA sequences that encode said peptides without appreciable loss of their biological utility or activity.

In many instances, a modified polypeptide will contain one or more conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged.

In making such changes, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i.e.* still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively on the basis of hydrophilicity. U. S. Patent 4,554,101 states that the greatest local average hydrophilicity of a protein, as governed by the hydrophilicity of its adjacent amino acids, correlates with a biological property of the protein.

As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A modified polypeptide may also, or alternatively, contain nonconservative changes. In a preferred embodiment, variant polypeptides differ from a native sequence by substitution, deletion or addition of five amino acids or fewer. Modified polypeptides may also (or alternatively) be modified by, for example, the deletion or addition of amino acids that have minimal influence on the immunogenicity, secondary structure and hydropathic nature of the polypeptide.

Polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide (*e*.*g*., poly-His), or to enhance binding of the polypeptide to a solid support. For example, a polypeptide may be conjugated to a biotin, streptavidin, or Fc immunoglobulin.

When comparing polynucleotide and polypeptide sequences, two sequences are said to be "identical" if the sequence of nucleotides or amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods. in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, optimal alignment of sequences for comparison may be conducted by the local identity algorithm of Smith and Waterman (1981) Add. APL. Match 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One non-limiting example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the disclosure. Software for performing BLAST analyses is publicly available through the National Center for Biotechnology Information.

In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Natl. Acad. Sci. USA 89:10915) alignments, (B) of 50, expectation (E) of 10, M=5, N=-4 and a comparison of both strands.

For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In one approach, the "percentage of sequence identity" is determined by comparing two optimally aligned sequences over a window of comparison of at least 20 positions, wherein the portion of the polynucleotide or polypeptide sequence in the comparison window may comprise additions or deletions *(i.e.,* gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residues occur in both sequences to yield the number of matched positions, dividing the number of matched positions by the total number of positions in the reference sequence (*i.e.,* the window size) and multiplying the results by 100 to yield the percentage of sequence identity.

"Homology" refers to the percentage of residues in the polynucleotide or polypeptide sequence variant that are identical to the non-variant sequence after aligning the sequences and introducing gaps, if necessary, to achieve the maximum percent homology. In particular embodiments, polynucleotide and polypeptide variants have at least 70%, at least 75%, at least 80%, at least 90%, at least 95%, at least 98%, or at least 99% polynucleotide or polypeptide homology with a polynucleotide or polypeptide described herein.

It will be appreciated by those of ordinary skill in the art that, as a result of the degeneracy of the genetic code, there are multiple nucleotide sequences that encode a polypeptide as described herein. Some of these polynucleotides bear minimal homology to the nucleotide sequence of any native gene. Nonetheless, polynucleotides that encode a polypeptide of the present disclosure but which vary due to differences in codon usage are specifically contemplated by the disclosure. Further, alleles of the genes including the polynucleotide sequences provided herein are within the scope of the disclosure. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

In certain embodiments of the present disclosure, mutagenesis of the disclosed polynucleotide sequences is performed in order to alter one or more properties of the encoded polypeptide, such as its binding specificity or binding strength. Techniques for mutagenesis are well-known in the art, and are widely used to create variants of both polypeptides and polynucleotides. A mutagenesis approach, such as site-specific mutagenesis, is employed for the preparation of variants and/or derivatives of the polypeptides described herein. By this approach, specific modifications in a polypeptide sequence are made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences include the nucleotide sequence of the desired mutation, as well as a sufficient number of adjacent nucleotides, to provide a primer sequence of sufficient size and sequence complexity to form a stable duplex on both sides of the deletion junction being traversed. Mutations are employed in a selected polynucleotide sequence to improve, alter, decrease, modify, or otherwise change the properties of the polynucleotide itself, and/or alter the properties, activity, composition, stability, or primary sequence of the encoded polypeptide.

In other embodiments of the present disclosure, the polynucleotide sequences provided herein are used as probes or primers for nucleic acid hybridization, e.g., as PCR primers. The ability of such nucleic acid probes to specifically hybridize to a sequence of interest enables them to detect the presence of complementary sequences in a given sample. However, other uses are also encompassed by the disclosure, such as the use of the sequence information for the preparation of mutant species primers, or primers for use in preparing other genetic constructions. As such, nucleic acid segments of the disclosure that include a sequence region of at least about a 15-nucleotide long contiguous sequence that has the same sequence as, or is complementary to, a 15 nucleotide long contiguous sequence disclosed herein is particularly useful. Longer contiguous identical or complementary sequences, e.g., those of about 20, 30, 40, 50, 100, 200, 500, 1000 (including all intermediate lengths) including full length sequences, and all lengths in between, are also used in certain embodiments. The disclosure thus includes use of disclosed sequences in the design and preparation of nucleic acid primers and probes, such as for use in nucleic acid amplification and detection as non-limiting examples. In some embodiments, the primers may be used for nucleic acid sequencing to detect a disclosed sequence, such as a mutant sequence.

Polynucleotide molecules having sequence regions consisting of contiguous nucleotide stretches of 10-14, 15-20, 30, 50, or even of 100-200 nucleotides or so (including intermediate lengths as well), identical or complementary to a polynucleotide sequence disclosed herein, are particularly contemplated as hybridization probes for use in, *e.g.,* Southern and Northern blotting, and/or primers for use in, *e.g.,* polymerase chain reaction (PCR), quantitative PCR, or real-time PCR. The total size of fragment, as well as the size of the complementary stretch (es), ultimately depends on the intended use or application of the particular nucleic acid segment. Smaller fragments are generally used in hybridization embodiments, wherein the length of the contiguous complementary region may be varied, such as between about 15 and about 100 nucleotides, but larger contiguous complementarity stretches may be used, according to the length complementary sequences one wishes to detect.

The use of a hybridization probe of about 15-25 nucleotides in length allows the formation of a duplex molecule that is both stable and selective. Molecules having contiguous complementary sequences over stretches greater than 12 bases in length are generally preferred, though, in order to increase stability and selectivity of the hybrid, and thereby improve the quality and degree of specific hybrid molecules obtained. Nucleic acid molecules having gene-complementary stretches of 15 to 25 contiguous nucleotides, or even longer where desired, are generally preferred.

Hybridization probes are selected from any portion of any of the sequences disclosed herein. All that is required is to review the sequences set forth herein, or to any continuous portion of the sequences, from about 15-25 nucleotides in length up to and including the full length sequence, that one wishes to utilize as a probe or primer. The choice of probe and primer sequences is governed by various factors. For example, one may wish to employ primers from towards the termini of the total sequence.

Polynucleotide of the present disclosure, or fragments or modified sequences thereof, are readily prepared by, for example, directly synthesizing the fragment by chemical means, as is commonly practiced using an automated oligonucleotide synthesizer. Also, fragments are obtained by application of nucleic acid reproduction technology, such as the PCR™ technology of U. S. Patent 4,683,202, by introducing selected sequences into recombinant vectors for recombinant production, and by other recombinant DNA techniques generally known to those of skill in the art of molecular biology.

In some embodiments, polynucleotides as disclosed herein may be prepared for sequence determination or detection by any method known to the skilled person. Non-limiting examples include sequencing based on 1) reversible dye-terminators and attachment of DNA molecules to primers on a slide with amplification using four types of reversible terminator bases to extend the DNA only one nucleotide at a time followed by removal of the the dye along with the terminal 3' blocker to allow the next cycle of extension; 2) ligation of immobilized oligonucleotides of known sequences followed by PCR (optionally emulsion PCR) and sequencing; 3) hydrogen ion release due to nucleotide extension with detection by semiconductor; 4) nanoball sequencing; 5) addition of polyA tail adapters followed by nucleotide extension as sequencing; 6) single molecule real time (SMRT) sequencing by use of immobilized polymerase; 7) massively parallel signature sequencing (MPSS); 8) Polony sequencing; 9) pyrosequencing via single DNA templates hybridized to single primer coated beads; and 10) RNA polymerase (RNAP) mediated sequencing.

The analyses of the disclosure may be preceded or followed by a variety of related actions. In some embodiments, an analysis is preceded by a determination or diagnosis of a human subject as in need of the analysis. The analysis may be preceded by a determination of a need for the analysis, such as that by a medical doctor, nurse or other health care provider or professional, or those working under their instruction, or personnel of a health insurance or maintenance organization in approving the performance of the measurement as a basis to request reimbursement or payment for the performance. In some embodiments, an analysis may be followed by payment for performance of a disclosed method.

The analyses of the disclosure may also be preceded by preparatory acts necessary to an actual analysis. Non-limiting examples include the actual obtaining of a cell containing or nucleic acid containing or polypeptide containing sample from a human subject; or receipt of such a sample; or sectioning a cell containing sample; or isolating cells from a cell containing sample; or preparing nucleic acid molecules from cells of a cell containing sample; or reverse transcribing RNA from cells of a cell containing sample.

The disclosure further provides kits for the practice of any disclosed method as described herein. A kit will typically comprise one or more reagents to detect nucleic acid sequence or polypeptide sequence as described herein for the practice of the present disclosure. Non-limiting examples include polynucleotide probes or primers for the detection of expression levels, one or more enzymes used in the methods of the disclosure, and one or more containers or solid supports or solid medium for use in the practice of the disclosure. In some embodiments, the kit will include an array or other solid media, including a bead as a non-limiting example, for the detection of sequences as described herein. In other embodiments, the kit may comprise one or more antibodies that are immunoreactive with epitopes present on a polypeptide which indicates the presence of a gene sequence alteration as disclosed herein. In some embodiments, the antibody will be an antibody fragment.

A kit of the disclosure may also include instructional materials disclosing or describing the use of the kit or a primer or probe of the present disclosure in a method of the disclosure as provided herein. A kit may also include additional components to facilitate the particular application for which the kit is designed. Thus, for example, a kit may additionally contain means of detecting the label (e.g. enzyme substrates for enzymatic labels, filter sets to detect fluorescent labels, appropriate secondary labels such as a sheep anti-mouse-HRP, or the like). A kit may additionally include buffers and other reagents recognized for use in a method of the disclosure. In some embodiments, a kit may be designed for use as an *in vitro* diagnostic.

Having now generally provided the disclosure, the same will be more readily understood through reference to the following examples which are provided by way of illustration, and are not intended to be limiting of the disclosure, unless specified.

### EXAMPLES

### Example 1: Patients and Methods

### Patients

The study population included three clinical cohorts representative of different disease phases: *i*) fludarabine-refractory CLL (n=59), including cases (n=11) subjected to whole exome sequencing (Table 1); *ii)* a consecutive series of newly diagnosed and previously untreated CLL (n=301) (Table 2); and *iii)* clonally related RS (n=33; all diffuse large B cell lymphomas) (Table 3). CLL diagnosis was based on the IWCLL-NCI criteria (Hallek M, et al. Blood. 2008; 111(12):5446-5456); diagnosis of fludarabine-refractoriness was according to guidelines (Hallek M, et al. Blood. 2008; 111(12):5446-5456); RS was based on histological criteria (Muller-Hermelink HK, et al. Swerdlow SH et al eds. World Health Organization Classification of Tumours, Pathology and Genetics of Tumours of Haematopoietic and Lymphoid Tissues. Lyon, France: IARC; 2008:180-182; Stein H et al. Swerdlow SH et al. eds. World Health Organization Classification of Tumours, Pathology and Genetics of Tumours of Haematopoietic and Lymphoid Tissues. Lyon, France: IARC; 2008:233-237). Peripheral blood tumor samples were obtained as follows: *i*) for fludarabine-refractory CLL, immediately before starting the treatment to which the patient eventually failed to respond; *ii)* for newly diagnosed and previously untreated CLL, at disease presentation. All RS studies were performed on RS diagnostic biopsies. Normal DNAs from the same patients were obtained from saliva or from purified granulocytes and confirmed to be tumor-free by PCR of tumor-specific *IGHV-D-J* rearrangements. Patients provided informed consent in accordance with local IRB requirements and the Declaration of Helsinki. The study was approved by the Ethical Committee of the Ospedale Maggiore della Carità di Novara associated with the Amedeo Avogadro University of Eastern Piedmont (Protocol Code 59/CE; Study Number CE 8/11).

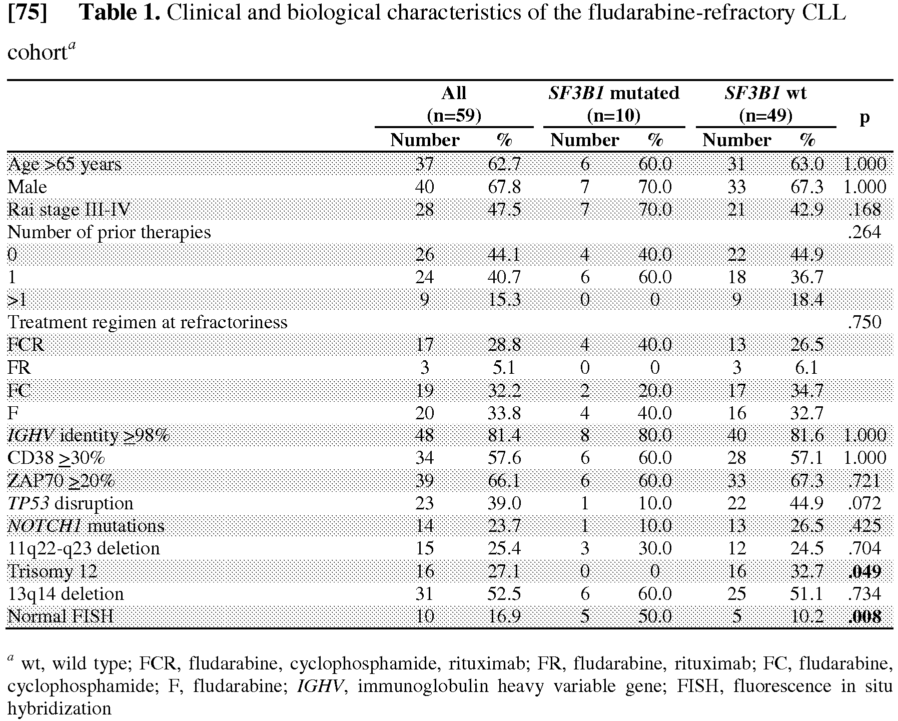

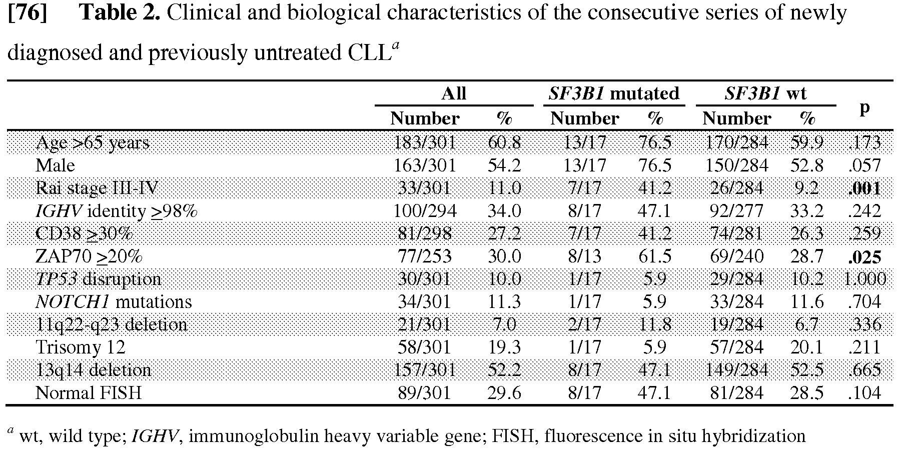

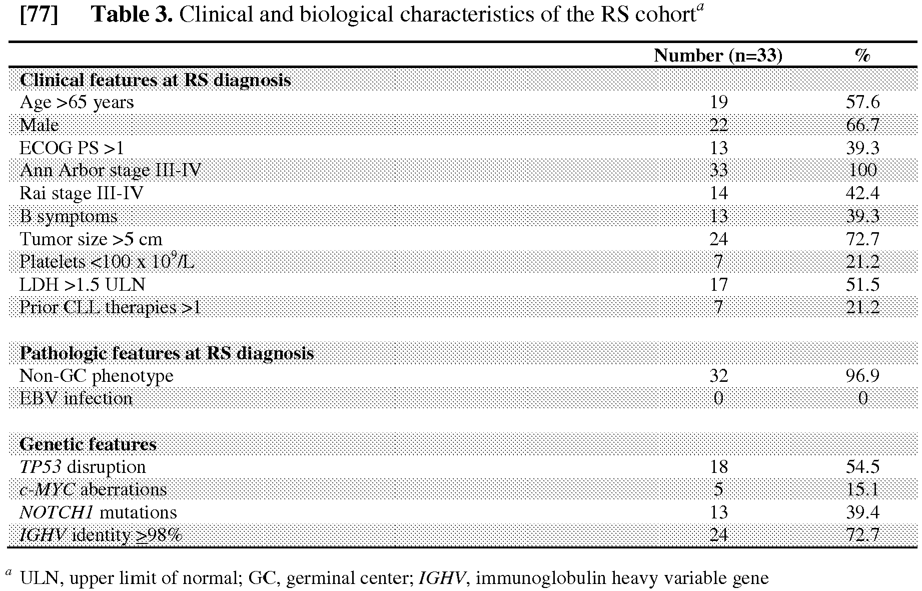

### Mutation analysis of SF3B1

Mutational analysis of *SF3B1* (exons 1-25, including splicing sites; RefSeq or Genbank Accession No. NM_012433.2) was performed on PCR amplimers obtained from genomic DNA by a combination of Sanger sequencing (performed on an ABI PRISM 3100 Genetic Analyzer, Applied Biosystems) and targeted next generation sequencing (performed on a Genome Sequencer Junior, 454 Life Sciences, Roche, Branford, CT; mean coverage ~200X). Sanger sequences were compared to the corresponding germline RefSeq using Mutation Surveyor Version 2.41 (SoftGenetics, State College, PA) after both automated and manual curation. Sequencing reads obtained by next generation sequencing were mapped on RefSeq using the Amplicon Variant Analyzer software package (Roche). All sequence variants identified by Sanger sequencing or next generation sequencing were subsequently confirmed by Sanger sequencing from both strands on independent amplimers. Synonymous mutations, germline polymorphisms known from databases (dbSNP132, Ensembl Database, UCSC Genome Browser), and changes present in matched normal DNA were removed from the analysis. Molecular studies were performed in blind with respect to clinical data. The prediction of functional effects of the amino acid substitutions was performed by using the PolyPhen-2 algorithm (Software version 2.1, genetics.bwh.harvard.edu/pph2) (Adzhubei IA, et al. Nat Methods. 2010; 7(4)248-249).

### Analysts of FISH karyotype and of IGHV, TP53 and NOTCH1 mutations

FISH analysis was performed as reported using probes LSI13 and LSID13S319, CEP12, LSIp53, and LSIATM (Abbott, Rome, Italy) (Rossi D, et al. Clin Cancer Res. 2009; 15(3):995-1004). *IGHV* mutational status was investigated as previously reported (Rossi D, et al. Clin Cancer Res. 2009; 15(13):4415-4422). Sequences were aligned to the ImMunoGeneTics sequence directory and considered mutated if identity to corresponding germline genes was <98% (Hamblin TJ, et al. Blood. 1999; 94(6):1848-1854; Damle RN, et al. Blood. 1999; 94(6):1840-1847). *TP53* and *NOTCH1* mutations were analyzed as reported (Rossi D, et al. Clin Cancer Res. 2009; 15(3):995-1004; Fabbri G, et al. J Exp Med. 2011; 208(7):1389-1401).

### Copy number analysis.

Genome-wide DNA profiles were obtained from high molecular weight genomic DNA of CLL patients using the Affymetrix Genome-Wide Human SNP Array 6.0 (Affymetrix, Santa Clara, CA, USA), following the manufacturer's instructions. The bioinformatics pipeline used for the identification of copy number alterations was previously described (Pasqualucci L, et al. Nat Genet. 2011; doi: 10.1038/ng.892; Rinaldi A, et al. Br J Haematol. 2011; doi: 10.1111/j.1365-2141.2011.08789.x).

### Gene expression profile analysis.

Gene expression profile analysis of purified normal B cell subpopulations and CLL samples was performed using Affymetrix HG-U133_plus2 arrays as part of an independent study (GEO database GSE12195). The probes used in Fig. 2D are the following: 228758_at, 203140_at, and 215990_s_at (for *BCL6*); 219841_at and 224499_s_at (for *AICDA);* 203684_s_at and 203685_at (for *BCL2*); 204562_at and 216986_s_at (for *IRF4*); and 201070_x_at, 201071_x_at, 211185_s_at, and 214305_s_at (for *SF3B1*).

### Statistical analysis

Overall survival was measured from date of diagnosis to date of death (event) or of last follow-up (censoring). Treatment free survival was measured from date of diagnosis to date of progressive and symptomatic disease requiring treatment according to IWCLL-NCI guidelines (event), death, or last follow up (censoring) (Hallek M, et al. Blood. 2008; 111(12):5446-5456). Survival was estimated by the Kaplan-Meier method (Kaplan EL and Meier P. Am Stat Assoc. 1958; 53:457-481). The crude association between *SF3B1* mutations and survival was estimated by log-rank analysis (Kaplan EL and Meier P. Am Stat Assoc. 1958; 53:457-481).

Categorical variables were compared by chi-square test and exact tests when appropriate. All statistical tests were two-sided. Statistical significance was defined as p value <.05. The analysis was performed with the Statistical Package for the Social Sciences (SPSS) software v.18.0 (Chicago, IL).

### Example 2: Mutations in the SF3B1 Splicing Factor Affect Progression and Fludarabine-Refractoriness of Chronic Lymphocytic Leukemia

Following the initial observation of recurrent *SF3B1* mutations in 3/11 fludarabinerefractory CLL analyzed by whole exome sequencing, targeted re-sequencing of the *SF3B1* coding sequence and splice sites was performed in 48 additional cases of progressive and fludarabinerefractory CLL (total number of cases analyzed: 59), collected at the time of progression immediately before starting the treatment to which the patient eventually failed to respond (Table 1). *SF3B1* was altered in 10/59 (17%) fludarabine-refractory CLL by missense mutations (n=9) or in-frame deletions (n=1) clustering in the HEAT3, HEAT4 and HEAT5 repeats of the *SF3B1* protein (Fig. 1 and Fig. 2A; Table 4). Two sites that are highly conserved inter-species (codon 662 and codon 700) were recurrently mutated in 3 and 5 cases, respectively (Fig. 1). *SF3B1* mutations were monoallelic and were predicted to be functionally significant according to the PolyPhen-2 algorithm (Table 4) (Adzhubei IA, et al. Nat Methods. 2010; 7(4):248-249). These data document that mutations of *SF3B1,* a splicing factor that is a critical component of the spliceosome, recurrently associate with fludarabine-refractory CLL.

The biological characteristics of fludarabine-refractory CLL harboring *SF3B1* mutations are summarized in Table 1. Mutations occurred irrespective of the *IGHV* mutation status, CD38 expression and ZAP70 expression. At the time of fludarabine-refractoriness, *SF3B1* mutations were enriched in cases harboring a normal FISH karyotype (p=.008; Table 1). Also, *SF3B1* mutations distributed in a mutually exclusive fashion compared to *TP53* disruption tested by deletion and/or mutation (mutual information I =0.0609; p=.046; Fig. 2B). By combining *SF3B1* mutations with other genetic lesions enriched in chemorefractory cases *(TP53* disruption, *NOTCH1* mutations, *ATM* deletion) (Fabbri G, et al. J Exp Med. 2011; 208(7):1389-1401; Döhner H, et al. N Engl J Med. 2000; 343(26):1910-1916; Rossi D, et al. Clin Cancer Res. 2009; 15(3):995-1004; Zenz T, et al. Blood. 2009;114(13):2589-2597; Stilgenbauer S, et al. J Clin Oncol. 2009;27(24):3994-4001), fludarabine-refractory CLL appeared to be characterized by multiple molecular alterations that, to some extent, are mutually exclusive (Fig. 2B).

To investigate whether *SF3B1* mutations are restricted to chemorefractory cases, the prevalence of mutations observed at the time of fludarabine-refractoriness was then compared to the prevalence of mutations observed in other disease phases. In a consecutive series evaluated at CLL diagnosis, *SF3B1* mutations were rare (17/301; 5%) (Fig. 2A; Table 4), and showed a crude association with short treatment free survival (p<.001) and overall survival (p=.011) (Fig. 2C). Remarkably, 5/17 (29%) CLL mutated at diagnosis were primary fludarabine-refractory patients. One patient with wild type *SF3B1* alleles at diagnosis subsequently acquired a *SF3B1* mutation concomitant with the development of fludarabine-refractoriness (case 7915 in Table 4). In CLL investigated at diagnosis, the hot-spot distribution and molecular spectrum of *SF3B1* mutations, as well as their mutual relationship with other genetic lesions, were similar to those observed in fludarabine-refractory CLL (Fig. 1 and 2B; Table 4). *SF3B1* mutations were only found in 2/33 (6.0%) clonally-related RS (Fig. 1 and 2A; Table 4). Across the different disease phases investigated, mutations were confirmed to be somatically acquired in all cases (n=18) for which germline DNA was available (Table 4). Although the relative expression of *SF3B1* in CLL was higher compared to normal B-cell subsets (Figure 2D), extensive investigation by SNP array analysis ruled out focal copy number abnormalities of *SF3B1* in this leukemia (n=0/323). These data document that SF3B1 mutations: i) occur at a low rate at CLL presentation, whereas they are enriched in fludarabine-refractory cases; ii) play a minor role in RS transformation, corroborating the notion that CLL histologic shift is molecularly distinct from chemorefractory progression without RS transformation (Rossi D, et al. Blood. 2011; 117(12):3391-3401).

The identification of SF3B1 mutations points to the involvement of splicing regulation as a novel pathogenetic mechanism in CLL. SF3B1 is a critical component of both major (U2-like) and minor (U12-like) spliceosomes (Luke MM, et al. Mol Cell Biol. 1996; 16(6):2744-2755; Wang C, et al. Genes Dev. 1998; 12(10): 1409-1414; Das BK,et al. Mol Cell Biol. 1999; 19(10):6796-6802), which enact the precise excision of introns from pre-mRNA (Wahl MC, et al. Cell. 2009; 136(4):701-718; David CJ and Manley JL. Genes Dev. 2010; 24(21):2343-2364; Ward AJ and Cooper TA. J Pathol. 2010; 220(2): 152-163. The precise biological role of *SF3B1* mutations in CLL is currently elusive. The pathogenicity of *SF3B1* mutations in CLL is strongly supported by the clustering of these mutations in evolutionarily conserved hotspots localized within HEAT domains, which are tandemly arranged curlicue-like structures serving as flexible scaffolding on which other components can assemble (Andrade MA and Bork P. Nat Genet. 1995; 11(2): 115-116; Andrade MA, et al. J Struct Biol. 2001; 134(2-3):117-13 1). Also, the observation that SF3B1 regulates the alternative splicing program of genes controlling cell cycle progression and apoptosis points to a potential contribution of *SF3B1* mutations in modulating tumor cell proliferation and survival (David CJ and Manley JL. Genes Dev. 2010; 24(21):2343-2364; Kaida D, et al. Nat Chem Biol. 2007; 3(9):576-583; Corrionero A, et al. Genes Dev. 2011; 25(5):445-459).

In addition to pathogenetic implications, *SF3B1* mutations also provide a therapeutic target for *SF3B1* inhibitors (Kaida D, et al. Nat Chem Biol. 2007; 3(9):576-583; Corrionero A, et al. Genes Dev. 2011; 25(5):445-459), which are currently under pre-clinical development as anti-cancer drugs.

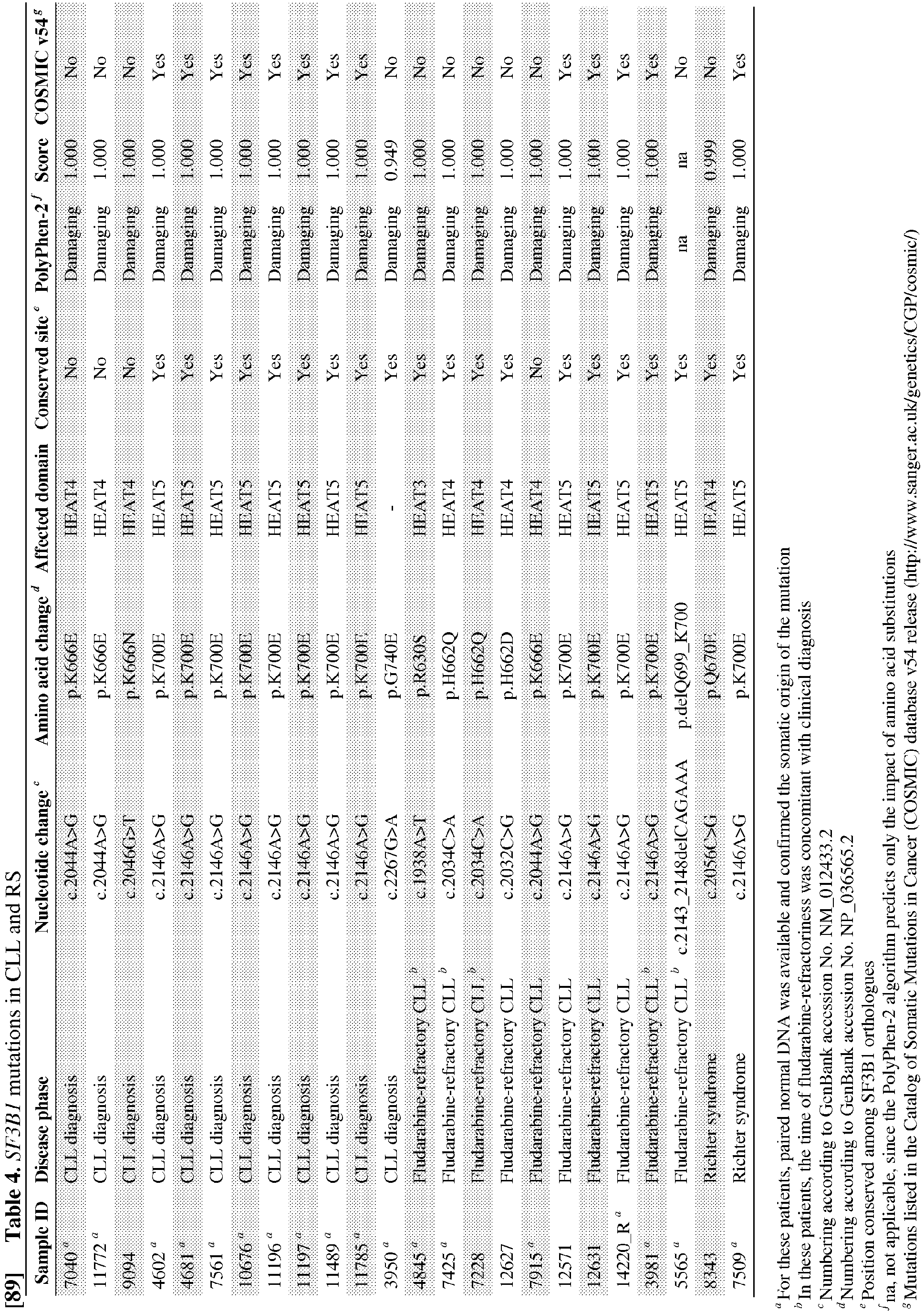

The citation of documents herein is not to be construed as reflecting an admission that any is relevant prior art. Moreover, their citation is not an indication of a search for relevant disclosures. All statements regarding the date(s) or contents of the documents is based on available information and is not an admission as to their accuracy or correctness.

## Claims

1. An *in vitro* method of prognosing a subject with chronic lymphocytic leukemia (CLL), comprising:
(a) determining the sequence of a portion of the *SF3B1* gene in a biological sample obtained from the subject, wherein the portion of the *SF3B1* gene comprises a sequence that encodes for a HEAT3, HEAT4 or HEAT5 domain; and
(b) analyzing the sequence of the *SF3B1* gene for a mutation,
wherein the presence of the mutation in the sequence of the *SF3B1* gene predicts a decreased survival of the subject,
thereby prognosing the subject with chronic lymphocytic leukemia (CLL).

2. An *in vitro* method of determining the response of a subject with chronic lymphocytic leukemia (CLL) to treatment with fludarabine, comprising:
(a) determining the sequence of a portion of the *SF3B1* gene in a biological sample obtained from the subject, wherein the portion of the *SF3B1* gene comprises a sequence that encodes for a HEAT3, HEAT4 or HEAT5 domain; and
(b) analyzing the sequence of the *SF3B1* gene for a mutation,
wherein the presence of the mutation in the sequence of the *SF3B1* gene indicates that the subject is resistant or refractory to fludarabine,
thereby determining the response of the subject with chronic lymphocytic leukemia (CLL) to treatment with fludarabine.

3. An *in vitro* method of prognosing a subject with chronic lymphocytic leukemia (CLL),
comprising:
(a) determining the sequence of a portion of the SF3B1 polypeptide in a biological sample obtained from the subject, wherein the portion of the SF3B1 polypeptide comprises the sequence of a HEAT3, HEAT4 or HEAT5 domain; and
(b) analyzing the sequence of the SF3B1 polypeptide for a mutation,
wherein the presence of the mutation in the sequence of the SF3B polypeptide predicts a decreased survival of the subject,
thereby prognosing the subject with chronic lymphocytic leukemia (CLL).

4. An *in vitro* method of determining the response of a subject with chronic lymphocytic leukemia (CLL) to treatment with fludarabine, comprising:
(a) determining the sequence of a portion of the SF3B1 polypeptide in a biological sample obtained from the subject, wherein the portion of the SF3B1 polypeptide comprises a sequence of a HEAT3, HEAT4 or HEAT5 domain; and
(b) analyzing the sequence of the SF3B1 polypeptide for a mutation,
wherein the presence of the mutation in the sequence of the SF3B polypeptide indicates that the subject is resistant or refractory to fludarabine,
thereby determining the response of the subject with chronic lymphocytic leukemia (CLL) to treatment with fludarabine.

5. The method of claim 1 or 3, wherein the decreased survival comprises treatment-free survival or overall survival.

6. The method of claim 4 wherein treatment with fludarabine is to be discontinued or is to be replaced by treatment with chlorambucil, cyclophosphamide, rituximab, alemtuzumab, bendamustine, or a combination thereof.

7. The method of claim 4, wherein the subject is to be treated with chlorambucil, cyclophosphamide, rituximab, alemtuzumab, bendamustine, or a combination thereof.

8. The method of any one of claims 1-4, wherein the analyzing step further comprises polymerase chain reaction (PCR), Sanger sequencing, next generation sequencing, or a combination thereof.

9. The method of any one of claims 1-4, wherein the subject has been diagnosed with chronic lymphocytic leukemia (CLL) and/or wherein the mutation is a missense mutation or an in-frame deletion.

10. The method of claim 1 or 2, wherein the biological sample comprises an isolated and purified genomic DNA, cDNA, or RNA molecule, or the method of claim 3 or 4 wherein the biological sample comprises an isolated and purified polypeptide molecule.

11. The method of claim 3 or 4, wherein the portion of the *SF3B1* polypeptide comprising the sequence of a HEAT3, HEAT4 or HEAT5 domain is selected from the group consisting of SEQ ID NO: 1, 2, 3, 4, 5, 6, 7, 8, 9, 10, 11, 12, 13, 14, 15, 16, and 19, and is preferably SEQ ID NO: 1 or 19.

12. The method of claim 1, wherein the mutation is:
a substitution of a Guanine (G) for an Adenine (A) at nucleotide base position 2044 or 2146 of SEQ ID NO: 17; or
a substitution of a Thymine (T) for a Guanine (G) at nucleotide base position 2046 of SEQ ID NO: 17; or
a substitution of an Adenine (A) for a Guanine (G) at nucleotide base position 2267 of SEQ ID NO: 17.

13. The method of claim 2, wherein the mutation is:
a substitution of a Thymine (T) for an Adenine (A) at nucleotide base position 1938 of SEQ ID NO: 17; or
a substitution of an Adenine (A) for a Cytosine (C) at nucleotide base position 2034 of SEQ ID NO: 17; or
a substitution of a Guanine (G) for a Cytosine (C) at nucleotide base position 2032 of SEQ ID NO: 17; or
a substitution of a Guanine (G) for an Adenine (A) at nucleotide base position 2044 of SEQ ID NO: 17; or
a substitution of a Guanine (G) for an Adenine (A) at nucleotide base position 2146 of SEQ ID NO: 17; or
a deletion of the nucleotide sequence CAGAAA corresponding to nucleotide base positions 2143 to 2148 of SEQ ID NO: 17; or
a substitution of a Guanine (G) for a Cytosine (C) at nucleotide base position 2056 of SEQ ID NO: 17.

14. The method of claim 3, wherein the mutation results in:
a substitution of a Glutamic Acid (Glu or E) residue for a Lysine (Lys or K) residue at codon 666 or 700 of SEQ ID NO: 19; or
a substitution of an Asparagine (Asn or N) residue for a Lysine (Lys or K) residue at codon 666 of SEQ ID NO: 19; or
a substitution of a Glutamic Acid (Glu or E) residue for a Glycine (Gly or G) residue at codon 740 of SEQ ID NO: 19.

15. The method of claim 4, wherein the mutation results in:
a substitution of a Serine (Ser or S) residue for an Arginine (Arg or R) residue at codon 630 of SEQ ID NO: 19; or
a substitution of a Glutamine (Gln or Q) residue for a Histidine (His or H) residue at codon 662 of SEQ ID NO: 19; or
a substitution of an Aspartic Acid (Asp or D) residue for a Histidine (His or H) residue at codon 662 of SEQ ID NO: 19; or
a substitution of a Glutamic Acid (Glu or E) residue for a Lysine (Lys or K) residue at codon 666 or 700 of SEQ ID NO: 19; or
a deletion of a Glutamine (Gln or Q) residue at codon 699 and a Lysine (Lys or K) residue at codon 700 of SEQ ID NO: 19; or
a substitution of a Glutamic Acid (Glu or E) residue for a Glutamine (Gln or Q) residue at codon 670 of SEQ ID NO: 19.

## Patentansprüche

1. *In*-*vitr*o-Verfahren zum Erstellen einer Prognose für ein Subjekt mit chronischer lymphozytärer Leukämie (chronic lymphocytic leukemia (CLL)), umfassend:
(a) Bestimmen der Sequenz eines Teils des *SF3B1-Gens* in einer biologischen Probe, die von dem Subjekt erhalten worden war, wobei der Teil des *SF3B1-Gens* eine Sequenz umfasst, die für eine HEAT3-, HEAT4- oder HEAT5-Domäne codiert und
(b) Analysieren der Sequenz des *SF3B1-Gens* auf eine Mutation,
wobei das Vorliegen der Mutation in der Sequenz des *SF3B1-Gens* ein verringertes Überleben des Subjekts voraussagt, wodurch eine Prognose für das Subjekt mit chronischer lymphozytärer Leukämie (CLL) erstellt wird.

2. *In*-*vitro*-Verfahren zum Bestimmen des Ansprechens eines Subjekts mit chronischer lymphozytärer Leukämie (CLL) auf eine Behandlung mit Fludarabin, umfassend:
(a) Bestimmen der Sequenz eines Teils des *SF3B1-Gens* in einer biologischen Probe, die von dem Subjekt erhalten worden war, wobei der Teil des *SF3B1-Gens* eine Sequenz umfasst, die für eine HEAT3-, HEAT4- oder HEAT5-Domäne codiert und
(b) Analysieren der Sequenz des *SF3B1-Gens* auf eine Mutation,
wobei das Vorliegen der Mutation in der Sequenz des *SF3B1-Gens* anzeigt, dass das Subjekt gegenüber Fludarabin resistent oder refraktär ist,
wodurch das Ansprechen des Subjekts mit chronischer lymphozytärer Leukämie (CLL) auf Behandlung mit Fludarabin bestimmt wird.

3. *In-vitro*-Verfahren zum Erstellen einer Prognose für ein Subjekt mit chronischer lymphozytärer Leukämie (CLL),
umfassend:
(a) Bestimmen der Sequenz eines Teils des *SF3B1*-Polypeptids in einer biologischen Probe, die von dem Subjekt erhalten worden war, wobei der Teil des *SF3B]*-Polypeptids die Sequenz einer HEAT3-, HEAT4- oder HEAT5-Domäne umfasst und
(b) Analysieren der Sequenz des *SF3B1*-Polypeptids auf eine Mutation,
wobei das Vorliegen der Mutation in der Sequenz des *SF3B1*-Polypeptids ein verringertes Überleben des Subjekts voraussagt,
wodurch eine Prognose für das Subjekt mit chronischer lymphozytärer Leukämie (CLL) erstellt wird.

4. *In*-*vitro*-Verfahren zum Bestimmen des Ansprechens eines Subjekts mit chronischer lymphozytärer Leukämie (CLL) auf eine Behandlung mit Fludarabin, umfassend:
(a) Bestimmen der Sequenz eines Teils des *SF3B1*-Polypeptids in einer biologischen Probe, die von dem Subjekt erhalten worden war, wobei der Teil des *SF3B1*-Polypeptids eine Sequenz einer HEAT3-, HEAT4- oder HEAT5-Domäne umfasst und
(b) Analysieren der Sequenz des *SF3B1*-Polypeptids auf eine Mutation,
wobei das Vorliegen der Mutation in der Sequenz des *SF3B1*-Polypeptids anzeigt, dass das Subjekt gegenüber Fludarabin resistent oder refraktär ist,
wodurch das Ansprechen des Subjekts mit chronischer lymphozytärer Leukämie (CLL) auf Behandlung mit Fludarabin bestimmt wird.

5. Verfahren gemäß Anspruch 1 oder 3, wobei das verringerte Überleben ein behandlungsfreies Überleben oder Gesamtüberleben umfasst.

6. Verfahren gemäß Anspruch 4, wobei eine Behandlung mit Fludarabin abzubrechen ist oder durch eine Behandlung mit Chlorambucil, Cyclophosphamid, Rituximab, Alemtuzumab, Bendamustin oder einer Kombination davon zu ersetzen ist.

7. Verfahren gemäß Anspruch 4, wobei das Subjekt mit Chlorambucil, Cyclophosphamid, Rituximab, Alemtuzumab, Bendamustin oder einer Kombination davon zu behandeln ist.

8. Verfahren gemäß einem der Ansprüche 1 bis 4, wobei der Schritt des Analysierens außerdem Polymerase-Kettenreaktion (PCR), Sanger-Sequenzierung, Sequenzierung der nächsten Generation oder eine Kombination davon umfasst.

9. Verfahren gemäß einem der Ansprüche 1 bis 4, wobei bei dem Subjekt chronische lymphozytäre Leukämie (CLL) diagnostiziert worden ist und/oder wobei die Mutation eine Missense-Mutation oder eine "In-Frame"-Deletion ist.

10. Verfahren gemäß Anspruch 1 oder 2, wobei die biologische Probe ein isoliertes und gereinigtes genomisches DNA-, cDNA- oder RNA-Molekül umfasst oder Verfahren gemäß Anspruch 3 oder 4, wobei die biologische Probe ein isoliertes und gereinigtes Polypeptid-Molekül umfasst.

11. Verfahren gemäß Anspruch 3 oder 4, wobei der Teil des *SF3B1*-Polypeptids, das die Sequenz einer HEAT3-, HEAT4- oder HEAT5-Domäne umfasst, aus der Gruppe, bestehend aus SEQ ID NO: 1, 2, 3, 4, 5, 6, 7, 8, 9, 10, 11, 12, 13, 14, 15, 16 und 19 ausgewählt ist und vorzugsweise SEQ ID NO: 1 oder 19 ist.

12. Verfahren gemäß Anspruch 1, wobei die Mutation ist:
eine Substitution eines Guanin (G) für ein Adenin (A) an Nucleotid-Basen-Position 2044 oder 2146 von SEQ ID NO: 17 oder
eine Substitution eines Thymin (T) für ein Guanin (G) an Nucleotid-Basen-Position 2046 von SEQ ID NO: 17 oder
eine Substitution eines Adenin (A) für ein Guanin (G) an Nucleotid-Basen-Position 2267 von SEQ ID NO: 17.

13. Verfahren gemäß Anspruch 2, wobei die Mutation ist:
eine Substitution eines Thymin (T) anstelle eines Adenins (A) an Nucleotid-Basen-Position 1938 von SEQ ID NO: 17 oder
eine Substitution eines Adenin (A) anstelle eines Cytosins (C) an Nucleotid-Basen-Position 2034 von SEQ ID NO: 17 oder
eine Substitution eines Guanin (G) anstelle eines Cytosins (C) an Nucleotid-Basen-Position 2032 von SEQ ID NO: 17 oder
eine Substitution eines Guanin (G) anstelle eines Adenins (A) an Nucleotid-Basen-Position 2044 von SEQ ID NO: 17 oder
eine Substitution eines Guanin (G) anstelle eines Adenins (A) an Nucleotid-Basen-Position 2146 von SEQ ID NO: 17 oder
eine Deletion der Nucleotid-Sequenz CAGAAA, die Nucleotid-Basen-Positionen 2143 bis 2148 von SEQ ID NO: 17 entspricht oder
eine Substitution eines Guanin (G) anstelle eines Cytosins (C) an Nucleotid-Basen-Position 2056 von SEQ ID NO: 17.

14. Verfahren gemäß Anspruch 3, wobei die Mutation resultiert in:
einer Substitution eines Glutaminsäure (Glu oder E)-Restes anstelle eines Lysin (Lys oder K)-Rests an Codon 666 oder 700 von SEQ ID NO: 19 oder
einer Substitution eines Asparagin (Asn oder N)-Restes anstelle eines Lysin (Lys oder K)-Rests an Codon 666 von SEQ ID NO: 19 oder
einer Substitution eines Glutaminsäure (Glu oder E)-Restes anstelle eines Glycin (Gly oder G)-Rests an Codon 740 von SEQ ID NO: 19.

15. Verfahren gemäß Anspruch 4, wobei die Mutation resultiert in:
einer Substitution eines Serin (Ser oder S)-Restes anstelle eines Arginin (Arg oder R)-Rests an Codon 630 von SEQ ID NO: 19 oder
einer Substitution eines Glutamin (Gln oder Q)-Restes anstelle eines Histidin (His oder H)-Rests an Codon 662 von SEQ ID NO: 19 oder
einer Substitution eines Asparaginsäure (Asp oder D)-Restes anstelle eines Histidin (His oder H)-Rests an Codon 662 von SEQ ID NO: 19 oder
einer Substitution eines Glutaminsäure (Glu oder E)-Restes anstelle eines Lysin (Lys oder K)-Rests an Codon 666 oder 700 von SEQ ID NO: 19 oder
einer Deletion eines Glutamin (Gln oder Q)-Restes an Codon 699 und eines Lysin (Lys oder K)-Restes an Codon 700 von SEQ ID NO: 19 oder
einer Substitution eines Glutaminsäure (Glu oder E)-Restes anstelle eines Glutamin (Gln oder Q)-Rests an Codon 670 von SEQ ID NO: 19.

## Revendications

1. Procédé *in vitro* d'établissement d'un pronostic concernant un sujet atteint de leucémie lymphoïde chronique (LLC), comportant les étapes suivantes :
a) déterminer la séquence d'une partie du gène *SF3B1* dans un échantillon biologique prélevé chez le sujet, étant entendu que ladite partie du gène *SF3B1* comprend une séquence qui code un domaine HEAT3, HEAT4 ou HEAT5 ;
b) et analyser la séquence du gène *SF3B1* pour y rechercher une mutation,
étant entendu que la présence d'une mutation dans la séquence du gène *SF3B1* permet de prédire une moindre durée de survie du sujet ;
ce qui fait que l'on établit un pronostic concernant le sujet atteint de leucémie lymphoïde chronique (LLC).

2. Procédé *in vitro* de détermination de la réponse d'un sujet atteint de leucémie lymphoïde chronique (LLC) à un traitement par fludarabine, comportant les étapes suivantes :
a) déterminer la séquence d'une partie du gène *SF3B1* dans un échantillon biologique prélevé chez le sujet, étant entendu que ladite partie du gène *SF3B1* comprend une séquence qui code un domaine HEAT3, HEAT4 ou HEAT5 ;
b) et analyser la séquence du gène *SF3B1* pour y rechercher une mutation,
étant entendu que la présence d'une mutation dans la séquence du gène *SF3B1* indique que le sujet est résistant ou réfractaire à la fludarabine ;
ce qui fait que l'on détermine la réponse du sujet atteint de leucémie lymphoïde chronique (LLC) à un traitement par fludarabine.

3. Procédé *in vitro* d'établissement d'un pronostic concernant un sujet atteint de leucémie lymphoïde chronique (LLC), comportant les étapes suivantes :
a) déterminer la séquence d'une partie du polypeptide SF3B1 dans un échantillon biologique prélevé chez le sujet, étant entendu que ladite partie du polypeptide SF3B comprend une séquence d'un domaine HEAT3, HEAT4 ou HEAT5 ;
b) et analyser la séquence du polypeptide SF3B pour y rechercher une mutation,
étant entendu que la présence d'une mutation dans la séquence du polypeptide *SF3B1* permet de prédire une moindre durée de survie du sujet,
ce qui fait que l'on établit un pronostic concernant le sujet atteint de leucémie lymphoïde chronique (LLC).

4. Procédé *in vitro* de détermination de la réponse d'un sujet atteint de leucémie lymphoïde chronique (LLC) à un traitement par fludarabine, comportant les étapes suivantes :
a) déterminer la séquence d'une partie du polypeptide SF3B1 dans un échantillon biologique prélevé chez le sujet, étant entendu que ladite partie du polypeptide SF3B comprend une séquence d'un domaine HEAT3, HEAT4 ou HEAT5 ;
b) et analyser la séquence du polypeptide SF3B pour y rechercher une mutation,
étant entendu que la présence d'une mutation dans la séquence du polypeptide SF3B1 indique que le sujet est résistant ou réfractaire à la fludarabine,
ce qui fait que l'on détermine la réponse du sujet atteint de leucémie lymphoïde chronique (LLC) à un traitement par fludarabine.

5. Procédé conforme à la revendication 1 ou 3, dans lequel la moindre durée de survie est le temps de survie sans traitement ou le temps de survie global.

6. Procédé conforme à la revendication 4, dans lequel le traitement par fludarabine doit être interrompu ou remplacé par un traitement par chlorambucil, cyclophosphamide, rituximab, alemtuzumab ou bendamustine, ou par une combinaison de ceux-ci.

7. Procédé conforme à la revendication 4, dans lequel le sujet doit être traité par chlorambucil, cyclophosphamide, rituximab, alemtuzumab ou bendamustine, ou par une combinaison de ceux-ci.

8. Procédé conforme à l'une des revendications 1 à 4, dans lequel l'étape d'analyse comporte en outre une opération d'amplification en chaîne par polymérase (PCR), de séquençage de Sanger ou de séquençage nouvelle génération, ou une combinaison de telles opérations.

9. Procédé conforme à l'une des revendications 1 à 4, dans lequel on a diagnostiqué chez le sujet une leucémie lymphoïde chronique (LLC) et/ou dans lequel la mutation est une mutation faux-sens ou une délétion en cadre.

10. Procédé conforme à la revendication 1 ou 2, dans lequel l'échantillon biologique comprend une molécule d'ADN génomique, d'ADNc ou d'ARN isolé et purifié, ou procédé conforme à la revendication 3 ou 4, dans lequel l'échantillon biologique comprend une molécule de polypeptide isolé et purifié.

11. Procédé conforme à la revendication 3 ou 4, dans lequel la partie du polypeptide SF3B comprenant une séquence d'un domaine HEAT3, HEAT4 ou HEAT5 est choisie dans l'ensemble formé par les Séquences N° 1, N° 2, N° 3, N° 4, N° 5, N° 6, N° 7, N° 8, N° 9, N° 10, N° 11, N° 12, N° 13, N° 14, N° 15, N° 16, et N° 19, et de préférence, est la Séquence N° 1 ou la Séquence N° 19.

12. Procédé conforme à la revendication 1, dans lequel la mutation est :
- le remplacement d'une adénine (A) par une guanine (G) en position de base de nucléotide n° 2044 ou 2146 dans la Séquence N° 17,
- le remplacement d'une guanine (G) par une thymine (T) en position de base de nucléotide n° 2046 dans la Séquence N° 17,
- ou le remplacement d'une guanine (G) par une adénine (A) en position de base de nucléotide n° 2267 dans la Séquence N° 17.

13. Procédé conforme à la revendication 1, dans lequel la mutation est :
- le remplacement d'une adénine (A) par une thymine (T) en position de base de nucléotide n° 1938 dans la Séquence N° 17,
- le remplacement d'une cytosine (C) par une adénine (A) en position de base de nucléotide n° 2034 dans la Séquence N° 17,
- le remplacement d'une cytosine (C) par une guanine (G) en position de base de nucléotide n° 2032 dans la Séquence N° 17,
- le remplacement d'une adénine (A) par une guanine (G) en position de base de nucléotide n° 2044 dans la Séquence N° 17,
- le remplacement d'une adénine (A) par une guanine (G) en position de base de nucléotide n° 2146 dans la Séquence N° 17,
- la délétion de la séquence de nucléotides CAGAAA correspondant aux positions de base de nucléotide n° 2143 à 2148 dans la Séquence N° 17,
- ou le remplacement d'une cytosine (C) par une guanine (G) en position de base de nucléotide n° 2056 dans la Séquence N° 17.

14. Procédé conforme à la revendication 1, dans lequel la mutation a pour résultat :
- le remplacement d'un résidu de lysine (Lys ou K) par un résidu d'acide glutamique (Glu ou E) au niveau du codon n° 666 ou 700 dans la Séquence N° 19,
- le remplacement d'un résidu de lysine (Lys ou K) par un résidu d'asparagine (Asn ou N) au niveau du codon n° 666 dans la Séquence N° 19,
- ou le remplacement d'un résidu de glycine (Gly ou G) par un résidu d'acide glutamique (Glu ou E) au niveau du codon n° 740 dans la Séquence N° 19.

15. Procédé conforme à la revendication 1, dans lequel la mutation a pour résultat :
- le remplacement d'un résidu d'arginine (Arg ou R) par un résidu de sérine (Ser ou S) au niveau du codon n° 630 dans la Séquence N° 19,
- le remplacement d'un résidu d'histidine (His ou H) par un résidu de glutamine (Gln ou Q) au niveau du codon n° 662 dans la Séquence N° 19,
- le remplacement d'un résidu d'histidine (His ou H) par un résidu d'acide aspartique (Asp ou D) au niveau du codon n° 662 dans la Séquence N° 19,
- le remplacement d'un résidu de lysine (Lys ou K) par un résidu d'acide glutamique (Glu ou E) au niveau du codon n° 666 ou 700 dans la Séquence N° 19,
- la délétion d'un résidu de glutamine (Gln ou Q) au niveau du codon n° 699 et d'un résidu de lysine (Lys ou K) au niveau du codon n° 700 dans la Séquence N° 19,
- ou le remplacement d'un résidu de glutamine (Glu ou Q) par un résidu d'acide glutamique (Glu ou E) au niveau du codon n° 670 dans la Séquence N° 19.
